# EUROPEAN PATENT SPECIFICATION

(11) **EP 1 250 056 B1**
(45) Date of publication and mention of the grant of the patent: **30.08.2006**
(21) Application number: 01902185.6
(22) Date of filing: 25.01.2001
(51) Int. Cl.: A23K 1/14, A23K 1/16, A23K 1/175, A61K 47/12, A61K 47/18, A61P 31/04

(54) **COMPOSITION FOR INTESTINAL DELIVERY**
ZUSAMMENSETZUNG ZUR INTESTINALEN VERABREICHUNG
COMPOSITION POUR ADMINISTRATION INTESTINALE

(30) Priority: 27.01.2000 US 178318 P
(43) Date of publication of application: 23.10.2002
(73) Proprietor: Peros Systèmes Technologies Inc., Saint Nicolas Québec G7A 3W4 (CA)
(72) Inventor: VANDENBERG, Grant, William, St-Augustin-de-Desmaures, Québec G3A 1H9 (CA)
(74) Representative: Nargolwalla, Cyra
(86) International application number: PCT/CA2001/000073
(87) International publication number: WO 2001/054514

(56) References cited:
- WO-A-93/11799
- WO-A-97/21448
- WO-A-97/40702
- MC LEAN E ET AL.: "Gastrointestinal delivery of peptide and protein drugs to aquacultured teleosts" AQUACULTURE, vol. 177, no. 1-4, 1 July 1999 (1999-07-01), pages 231-247, XP000996280
- SCHEP L J ET AL: "Controlled release opportunities for oral peptide delivery in aquaculture" JOURNAL OF CONTROLLED RELEASE,NL,ELSEVIER SCIENCE PUBLISHERS B.V., AMSTERDAM, vol. 59, no. 1, 1 May 1999 (1999-05-01), pages 1-14, XP004166210 ISSN: 0168-3659
- LAVELLE E C ET AL.: "The processing of an orally administered protein antigen in the digestive tract of rainbow trout, Ocorhyncus mykiss" COMPARATIVE BIOCHEMISTRY AND PHYSIOLOGY, vol. 117, no. 2, 1 June 1997 (1997-06-01), pages 263-275, XP000996478
- BRETON B ET AL.: "Improved bioavailability of orally delivered peptides and polypeptides in teleost fish" JOURNAL OF APPLIED ICHTHYOLOGY, vol. 14, no. 3-4, 1 December 1998 (1998-12-01), pages 251-257, XP000995987
- VAN HOOGDALEM E J ET AL: "Intestinal drug absorption enhancement: an overview" PHARMACOLOGY AND THERAPEUTICS,GB,ELSEVIER, vol. 44, no. 3, 1989, pages 407-443, XP002086283 ISSN: 0163-7258
- BERNKOP-SCHNURCH A: "The use of inhibitory agents to overcome the enzymatic barrier to perorally administered therapeutic peptides and proteins" JOURNAL OF CONTROLLED RELEASE,NL,ELSEVIER SCIENCE PUBLISHERS B.V. AMSTERDAM, vol. 52, no. 1-2, 2 March 1998 (1998-03-02), pages 1-16, XP004113649 ISSN: 0168-3659

## Description

### BACKGROUND OF THE INVENTION

### (a) Field of the invention

The present invention relates to a composition and a use of the composition in the manufacture of a medicament for oral administration of physiological active products and intestinal delivery thereof. The physiological active products administered with the present invention allows to achieve a better systemic delivery and, immunologic induction, and has demonstrated improved nutritional, nutraceutical, and therapeutic capacities.

### (b) Description of Prior Art

The conventional route of therapy involving protein or peptide drugs is via parenteral administration (i.e., by injection). This is primarily due to the lack of absorption of such drugs through the gastrointestinal tract. However, injections are painful and sometimes difficult to administer relative to other dosage forms. Patient compliance is an important consideration as well since some of these drugs may require frequent administration to juvenile or geriactric patients. Oral delivery is preferable to injections for patient acceptance since it is less painful and more convenient for the patient. However, delivery of therapeutic polypeptides through the gastrointestinal (Gl) tract has a number of problems such as low pH in the stomach, proteolytic degradation of the drug in the small intestine, low absorption through the intestinal membrane, and limited stability of such formulations, especially as an aqueous solution, which are all potential barriers to absorption of polypeptides following oral administration.

Recent efforts to deliver polypeptides orally have focused on the use of absorption enhancers. This has led to the discovery that a suspension of sodium salicylate in an excess of an oil can enhance the absorption of human growth hormone from the Gl tract. While absorption is improved by this combination, the bioavailability is of only up to about 10-20% of the protein (with reference to intravenous), which is still quite low. As a result, larger amounts of proteins must be administered orally in order to provide the required therapeutic level of protein in the plasma. This is a particular problem with proteins and polypeptides which, even with the advent of biotechnology, are still of relatively limited availability and are complex chemical entities as well, and very expensive as a result. Additionally, the liquid or semi-solid compositions of the prior art are difficult to formulate or package into a dosage form for oral delivery.

Other aspects can be considered. In mammal, gastric protein digestion must be of minor importance, as there is no significant protein malabsorption in individuals with the complete gastric atrophy of pernicious anemia. The reservoir role of the stomach is of considerable importance, as this results in a sustained entry of protein into the duodenum after a meal and ensure thorough mixing with pancreatic juice. The combination of a low pH and peptic activity probably results in denaturation of most food proteins, exposing bonds susceptible to further hydrolysis within the small intestine.

The epithelia lining the gastrointestinal tract clearly act also as a "frontier" between the external environment and the internal milieu of the body. They contain selective physiological mechanisms to control partly the entry and exit of molecules from the body, acting in a sense as a "valve" with biochemical properties remarkably similar to those of the kidney, another organ with "output valve" properties, though the term "valve" belittles the active metabolic processes that exert this function. The intestinal epithelium does act to an important extent as a physical barrier, a remarkable function for a single layer of potentially fragile cells being bombarded with physically and chemically noxious ingested material. However, it must be recognized that it is not such an "absolute" barrier as it has often been assumed. Hence, older concepts that intact proteins simply cannot enter the circulation in health, that gastrointestinal entry to the body is invariably highly selective, and that particulate material is invariably excluded have to be discarded in favor of a much more circumspect and complex view of barrier function. As well as their incomplete physical barrier functions, the gastrointestinal epithelia act as immunologic barrier and enzymatic barriers and these rather complex mechanisms are central to, and inseparable from, considerations of absorption of proteins and peptides in intact form.

The enzymatic barriers to peptide and protein by various routes have been studied in the case of oral or enteral delivery, enzymatic degradation before (or during) absorption undoubtedly provides the critical obstacle to absorption of peptide drugs in small intestine and a major barrier to absorption of peptides and proteins throughout the gastrointestinal tract.

Hence, for pharmaceutical delivery of peptides via enteral routes, inhibition of proteases or peptidases or the invention of formulations that provide protection against such degradation are vital targets for attention, possibly more important than absorption-enhancement strategies. Although teleology should be avoided, the multiplicity of barrier mechanisms in the gastrointestinal tract may be an evolutionary reflection of the severe consequences of uncontrolled ingress of exogenous biologically active material. Hence, these mechanisms inevitably pose considerable difficulty for the invention of effective modes of delivery for peptide and protein drugs. The concept of a digestive surface with a protective function, suggests that brush border bound proteases and peptidases have a role as a "hydrolytic barrier" to complement the "physical barrier" and the "immunologic barrier" each with protective function, whereas the cytoplasmic peptidases have a (merely) digestive function. This model becomes even more relevant if large-scale passage through paracellular routes (driven by solvent drag) becomes proven so that luminal and surface hydrolysis (before paracellular passage) would be critical in minimizing entry of biologically active peptides from partial digestion of dietary protein to the body.

In principle, potential routes across epithelia can be divided into (a) transcellular (including carrier-mediated mechanisms and endocytotic mechanisms; also diffusive routes through either aqueous channels or lipid parts of the membrane) and (b) paracellular (including passage through "tight" junctions and through extrusion zones, both those created during natural cell turnover and those arising following physical and chemical injury).

It is of a general view that absorption of intact peptides or proteins from the diet is of negligible direct nutritional significance on account of the small quantities absorbed compared to amounts of free amino acids entering the circulation. However, there are two reasons why absorption of these molecules in intact form is, or may be, beneficial. First, antigen sampling via the M-cell route is a vital part of the natural process of acquisition of mucosal immunity. Second, this route can potentially be exploited for therapeutic purposes. Apart from the relative ease, safety, (e.g. sterile instruments are unnecessary), noninvasive nature, and patient acceptability of the oral route compared to parenteral and rectal routes, the enteral route may provide beneficially greater access to the liver (relevant in the case of insulin administration). On the other hand, entry of some intact peptides and proteins to the circulation may be detrimental, although factual proof of this is currently incomplete, and many claims of pathological consequences arising from food proteins or peptides are based on no more than anecdotal or subjective evidence. In spit of this reservation, gastrointestinal food allergy is now an accepted phenomenon, and absorption of intact proteins to gain access to subepithelial mast cells is part of the mechanism.

Potential biological effects of absorbed intact proteins are not, of course, restricted to immunologic effects.

Agents enhancing the intestinal absorption are of particular importance in the field of oral administration of biologically active peptides and proteins for different applications. A valid reservation about the use of enhancers relates to the fact that they are essentially membrane- or junction-damaging agents. Enhancers, especially with chronic use, are likely to have toxic effects and to promote the ingress of unwanted molecules. The tryptophan supplementation which can produce harmful effects by a drastic increase in paracellular permeability is an intriguing result. It is possible that some individuals are particularly sensitive to tryptophan.

The use of liposome entrapment to facilitate peptide and protein from degradation has been evaluated, and subsequent results have been found disappointing. Another approach to use the lipid-soluble route, i.e. transcellular diffusion, is to formulate the peptide or protein in a microemulsion. A great deal of effort has been spent to try to devise means for delivering insulin orally. Almost every effort had succeeded, but only to a very limited extent. A water-in-oil microemulsion containing cholesterol, lecithin, and a fatty acid in critical proportions simulating the composition of chylomicons has been developed. Unfortunately, the credibility of these findings was called into question when a subsequent batch of the formulation was found to be contaminated with glibenclamide.

From the literature, it is apparent that anal presentation of proteins results in greater absorption and tissue accumulation than does oral presentation of the same dose. The question therefore arises as to whether uptake by the oral route can be increased. The potential may exist to at least enhance oral uptake values to that obtained by rectal challenge. Several methods of protecting pharmacologically active peptides and proteins from the action of the gut have been considered, including enteric and similar protective coating, co-administration with antiacids and enzymes inhibitors and delivery within bacterial cells and live foods (bioencapsulation).

For example, oral absorption of HRP (horseradish peroxidase) increased when co-delivered to rainbow trout with soya bean trypsin inhibitor. In the same study, co-delivery of an artificial detergent, Mega-9, was found to almost double plasma HRP presence when compared to controls. The increased protein concentrations were attained due to gelatinisation of the mucus coat and consequent increase in protein-enterocyte interactions. Also, it might be that the integrity of the Gl epithelia was breached, allowing for transcellular uptake. Mega-9, when orally intubated with recombinant bovine growth hormone (rbGH) and antacid, significantly increases growth performance of coho salmon over a 7-week trial period when compared to fish dosed with the growth hormone (GH) alone.

Detergent (L-lyso-phosphatidylcholine) have also been co-delivered with a gonadotropin releasing hormone agonists (GnRHA) during induced ovulation studies with sablefish *A. fimbria,* as a measure to maximize Gl absorption and elevate gastric pH, respectively. Oral intubation of goldfish with salmon pituitary extract and 0.2% polyacrylic acid produced antagonism.

The antibiotic monensin has been examined as an enhancing agent. The rationale underlying the selection of this compound was due to its ability to reduce transfer of pinocytosed proteins between vesicles and the lysosomal compartment. Furthermore, monensin had been demonstrated to raise the pH of acid compartments within various cell types. However, no effect upon HRP absorption was seen. The use of biodegradable microparticles to encapsulate, and thereby protect proteins from digestive processes, has been examined using complex protein macromolecules. In these studies, which employed HGG in Atlantic salmon *Salmo salar,* plasma presence of the protein was very much prolonged (up to 8 weeks) following oral intubation, suggesting that the entrapped HGG may have gained access via paracellular as well as transcellular pathways, resulting in sustained plasma residency. GH has also been delivered in a protected form, wherein the molecule was incorporated into a polymer matrix which remained intact under acidic conditions, but degraded under the alkaline conditions of the intestine. When added to food, the entrapped GH accelerated growth of rainbow trout over and above that seen for controls. An alternative method of protecting orally delivered pharmaceuticals, which has been used in the vaccination of fish, is with their delivery within another organism. With regard to bioactive proteins, this strategy has only been evaluated upon one occasion, using yeast recombinant for rainbow trout GH. In an elegant study, the feeding of striped mullet with diets supplemented with yeast recombinant for GH, resulted in significant growth rate of treated animals versus controls. This method, however, would be limited to those organisms, as exemplified by yeasts, which are able to store recombinant product, in an unmodified form. The recent past has also witnessed the development of a number of genetically engineered plants which express genes of pharmaceutical interest. Studies have demonstrated retained bioactivity of plant-based recombinant products when delivered orally. Similar methods for the administration of aquaculture-related production-oriented proteins would be highly attractive due to production economics. '

Developments in the biotechnology area have provided the means for producing virtually limitless supplies of bioactive peptides and proteins at economically viable levels. It is conceivable, therefore, that the natural permeability of animals, including human gut may be used as a means for delivering peptide and protein drugs to influence physiological control processes. Obvious oriented areas of application include controlled reproduction, growth acceleration, immune enhancement, therapeutical and nutritional improvement.

The oral route for vaccination offers significant advantage in that it reduces labor costs, is time-saving, decreases the possibilities for cross-contamination with needles and does not involve inventory handling or require disposal of treatment waters. With respect to the present review however, oral vaccination may only be considered based upon antigenic components. Trials with synthetic peptide-based virus vaccines in higher vertebrates have been reported to be successful although contradictory results have begun to emerge in large field-tests. For immunization programs, it is conceivable that recombinant subunit vaccines consisting of glycoproteins/nucleo-proteins could be incorporated into feeds.

A strategy that could control infections in all individuals would be any form of immunization that prevented or greatly reduced carriage, and hence transmission of microbials. In the case of immunization of young children with *Haemophilus influenzae,* for example, group b polysaccharide-protein conjugates, it has been observed that carriage is reduced from about 4% to less than 1%, a possible explanation of concomitant herd immunity. If a vaccine could prevent colonization by microbials, such a vaccine would be expected to prevent virtually all infections of a same gender of microbials in the immunized patients or animals. Since even unimmunized patients must acquire microbials from others, a vaccine that reduced carriage should reduce infections in immunocompromised, as well as unimmunized patients. In fact, an aggressive immunization program, coupled with antibiotic treatment of demonstrated carriers, might be able to eliminate the reservoir of this organism.

The principal determinant of specific immunity at mucosal surfaces is secretory IgA (S-lgA) which is physiologically and functionally separate from the components of the circulatory immune system. Mucosal S-lgA response are predominantly generated by the common mucosal immune system (CMIS) in which immunogens are taken up by specialized lympho-epithelial structures collectively referred to as musoca-associated lymphoid tissue (MALT).

Thus, immunization in the gut can elicit mucosal immunity in the upper airways and vice versa. The best-known MALT structures are the intestinal Peyer's patches. Further, it is important to note that oral immunization can induce an antigen-specific IgG response in the systemic compartment in addition to mucosal IgA antibodies.

Most soluble and non-replicating antigens are poor mucosal immunogens, especially by the peroral route, probably because digestive enzymes degrade them and have little or no tropism for the gut associated lymphoid tissue (GALT). A notable exception is cholera toxin (CT). CT is a potent mucosal immunogen probably because of the GM1 ganglioside-binding property of its binding subunit, CTB, that enables it to be taken up by the M cells of Peyer's patches and passed to the underlying immunocompetent cells. In addition to being a good mucosal immunogen, CT is an adjuvant that enhances the mucosal immunogenicity of other soluble antigens co-administered with it. Although it remains somewhat controversial, pure or recombinant CTB probably does not have these properties when administered intragastrically (i.g.) as an adjuvant. Very small amounts (<1mg) of intact CT, however, can act synergistically with CTB as an oral adjuvant. This finding may account for apparent adjuvant activity of many commercial preparations of CTB that usually contain small amounts of contaminating CT.

While the discovery of peptide compounds having nutritional and therapeutic value have moved rapidly in the last few years, the development of viable physiologically active agent delivery systems for many of these compounds has often proved problematic. The gastrointestinal tract secretes a variety of agents that metabolize polypeptides.

Exemplary of such catabolic agents are pepsin, trypsin, chymotrypsin, carboxypolypeptidases, aminopolypeptidases and dipeptidases. Polypeptides that escape catabolism in the stomach and small intestine are transported across the cells lining the gastrointestinal tract into the portal circulation, which carries absorbed polypeptides to the liver. Absorbed polypeptides are subject to further degradation by a myriad of hepatic metabolic events. Such hepatic degradation of absorbed materials from the blood before such materials enter the general systemic circulation is known in the pharmaceutical art as the "first pass effect".

Therefore, most, if not all, of these compounds must be administered parenterally as, for example, subcutaneous, intramuscular, or intraperitoneal injection. Since most patients cannot self-administer parenteral drug formulations, it is frequently necessary that drugs of this type be administered in an outpatient setting leading to additional costs associated with their use.

Working on the basis of the assumption that oral delivery of bioactive peptides and proteins to several animal species, as to farmed and aquacultured species for example, would provide benefit, it might be considered advantageous to be able to modify the uptake of pharmaceutical preparations.

There is, therefore, a pressing need for a new, efficient, cost effective and non-invasive method of administration to patients and animals of a composition containing nutritional and therapeutic agents, particularly peptides, which are otherwise unsuitable for oral administration.

### SUMMARY OF THE INVENTION

An object of the present invention is to provide a composition for oral administration to a human or an animal, including mammals, birds, insects, and fishes, for intestinal delivery of a physiologically active agent comprising, a neutralizing agent suitable for increasing gastric pH to between 6.5 to 9 in the animal digestive system to prevent the chemical denaturation, an inhibitor of digestive enzymes to prevent enzymatic digestion of the active agents, and an uptake increasing agent which increases intestinal absorption of a physiologically active agent. The invention is also based upon the finding that the combination of the three agents provides additive and synergistic intestinal delivery and uptake when used concurrently.

Another object of the present invention is to provide a use of the composition in the manufacture of a medicament for treating intestinal microbial infections in an animal, which comprises administrating 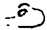 sufficient amount of the composition of the present invention, wherein the physiologically active agent is an antimicrobial agent.

In accordance with the present invention, there is provided a composition for oral administration to an animal for intestinal delivery of a physiologically action agent, the composition of the present invention comprises at least one neutralizing agent at concentration between about 1% to 60% w/w, an enzymatic inhibitor at concentration between about 1% to 50% w/w, and an uptake increasing agent at concentration between about 0.1 % to 50% w/w.

The composition according to the present invention, further comprises a physiologically active agent selected from the group consisting of therapeutical agents, nutritional products, mucopolysaccharides, lipids, carbohydrates, steroids, hormones, growth hormone (GH), growth hormone releasing hormone (GHRH), epithelial growth factor, vascular endothelial growth and permeability factor (VEGPF), nerve growth factor, cytokines, interleukins, interferons, GMCSF, hormone-like product, neurological factor, neurotropic factor, neurotransmitter, neuromodulator, enzyme, antibody, peptide, proteic fragment, vaccine, adjuvant, an antigen, immune stimulating or inhibiting factor, heomatopoietic factor, anti-cancer product, anti-inflammatory agent, anti-parasitic compound, anti-microbial agent, nucleic acid fragment, plasmid DNA vector, cell proliferation inhibitor or activator, cell differentiating factor, blood coagulation factor, immunoglobulin, anti-angiogenic product, negative selective markers or "suicide" agent, toxic compound, anti-angiogenic agent, polypeptide, anti-cancer agent, acid production drugs, and histamine H2-receptor antagonist.

The composition of the present invention comprises a neutralizing agent that is in amount sufficient to neutralize acidic degradation of the digestive system of the host animal and allow delivery of a physiologically active agent to the animal intestine, where the neutralizing agent may be selected from the group consisting of anti-acids, sodium bicarbonate, sodium carbonate, sodium citrate, sodium hydrogencarbonate, calcium phosphate, calcium carbonate, magnesium salts, magnesium carbonate, magnesium trisilicate, magnesium hydroxide, magnesium phosphate, magnesium oxide, bismuth subcarbonate, and combinations thereof.

The composition of the present invention may comprises a neutralizing agent which consists of at least one of sodium carbonate at a concentration of 10% to 20% w/w, and calcium carbonate at concentration of 10% to 20% w/w of the composition.

According to the present invention, there is provided a composition which comprises at least one enzyme inhibitor in an amount sufficient to substantially inhibit the degradation of a physiologically active agent by digestive enzymes in the digestive system of a human or an animal and allow delivery of this physiologically active agent into the intestine of the human or the animal.

The inhibitor of digestive enzymes may be selected from the group consisting of anti-proteases, egg albumin, plant-derived inhibitors from oilseeds, soybeans, kidney beans, faba beans, rice bran, wheat bran, ethylenediamine tetraacetate, alpha-1-antitrypsin, albumin, ovalbumin, and proteasomes.

The composition according to the present invention may comprises pepsin inhibitors, enteropeptidase inhibitors, and/or albumin at a concentration between 10% to 20% w/w.

The composition of the present invention may comprises an uptake increasing agent which may consists of bile salts, saponins, deoxycholate, sodium salicylate, sodium lauryl sulphate, oleic acid, linoleic acid, monoolein, lecithin, lysolecithin, polyoxyethylene sorbitan esters, p-t-octylphenoxypolyoxyethylene, N.lauryl-fi-D-maltopyranoside, 1-dodecylazacycloheptane-2-azone, and phospholipids.

The uptake-increasing agent may be the sodium deoxycholate at a concentration between 1 % to 5%.

The composition according to the invention may further comprises at least one additional ingredient selected from the group consisting of ethylenediamine tetraacetate, preservatives, actioxidants, colorants binders, tracers, one or more sweeteners, surfactants, unmoulding agents, flavouring agents, meals, beans, yeast, brewer yeast, mineral oil, vegetable oil, animal oil, lubricants, ointment, and combinations thereof.

Another object of the present invention is that physiologically active agent when delivered into the human or the animal intestine may be absorbed by the intestine for systemic delivery, or to have an effective physiological effect on intestinal wall.

Also, the composition according to the present invention may allow for a physiologically active agent when delivered into a human or an animal intestine to have a physiological effect into the content of the intestine. This application may further be used to stimulate the food transit throughout the gut, or to treat infectious diseases.

In accordance with the present invention, the physiologically active agent is capable of inducing mucosal immunity or systemic immune reaction in the host human or animal against mucosal infectious diseases. There is provided a use of the composition in the manufacture of a medicament for immunization of a host against mucosal microorganisms which comprises orally administering to the host an immunizing amount of microbial surface protein in the form of killed whole microorganisms, a lysate of microorganisms or an isolated antigen or an immunologic fragment thereof.

The present invention further provides a composition for oral administration to a host, preferably for administration into the gut (stomach, digestive tract) of a host to confer protection or elicit an immune response against microbial infections.

According to the present invention, a use of the composition in the manufacture of a medicament for treating intestinal microbial infections in an animal is provided, which comprises administrating the composition of the present invention comprising an anti-microbial agent in amount sufficient for therapeutic effectiveness.

The microbial infections may be caused by microorganisms selected from the group consisting of bacteria, mushrooms, yeasts, viruses, *Staphylococci, Streptococci, Micrococci, Peptococci, Peptostreptococci, Enterococci, Bacillus, Clostridium, Lactobacillus, Listeria, Erysipelothrix, Propionibacterium, Eubacterium, Corynobacterium, Mycoplasma, Ureaplasma, Streptomyces, Haemophilus, Nesseria, Eikenellus, Moraxellus, Actinobacillus, Pasteurella, Bacteroides, Fusobacteria, Prevotella, Porphyromonas, Veillonella, Treponema, Mitsuokella, Capnocytophaga, Campylobacter, Klebsiella, Chlamydia,* and Coliforms.

The antimicrobial agent used to treated microbial infections may be selected from the group consisting of antibiotics, bacteriocins, lantibiotics, probiotics, antifungics, antimycotics, antiparasitics, aminoglycosides, vancomycin, rifampin, lincomycin, chloramphenicol, and the fluoroquinol, penicillin, beta-lactams, amoxicillin, ampicillin, azlocillin, carbenicillin, mezlocillin, nafcillin, oxacillin, piperacillin, ticarcillin, ceftazidime, ceftizoxime, ceftriaxone, cefuroxime, cephalexin, cephalothin, imipenen, aztreonam, gentamicin, netilmicin, tobramycin, tetracyclines, sulfonamides, niacrolides, erythromicin, clarithromcin, azithromycin, polymyxin B, clindamycin antibiotic, and combinations thereof.

The invention is also to provide a use of the composition in the manufacture of a medicament for systemic delivery, which comprises oral administration to an animal of a therapeutical agent for treating a health disorder of the animal, which may further comprises an acceptable pharmaceutical carrier.

The composition of the present invention can be used in the manufacture of drugs or foods.

There is also provided according to the present invention enhancing intestinal uptake of human or an animal, which comprises administrating orally a physiologically effective amount of a physiologically active agent.

For the purpose of the present invention the following terms are defined below.

The term "therapeutic agent" is used in a generic sense and includes treating agents, prophylactic agents, and replacement agents, antimicrobial agents.

The term "common mucosal immune system" refers to the fact that immunization at any mucosal site can elicit an immune response at all other mucosal sites.

The terms "protein", "peptide" and "polypeptide" refer to both the naturally occurring chemical entities and the structurally similar bioactive equivalents derived from either endogenous, exogenous, or synthetic sources and is used to mean polymers of amino acids linked together by an amide type linkage known as a peptide bond.

The term "structurally similar bioactive equivalent" is meant a polypeptide with an amino acid sequence which, although not identical to that of the naturally occurring peptide, is sufficiently similar in structure to produce substantially equivalent therapeutic effects on the subject to that produced by the natural peptide itself.

The term "therapeutically effect amount" of a medicament is meant a sufficient amount of the compound to obtain the intended therapeutic benefit, at a reasonable benefit/risk ratio applicable to any medical treatment. It will be understood, however, that the total daily usage of the medicaments and compositions of the present invention will be decided by the attending physician with the scope of sound medical judgement. The specific therapeutically effective dose level for any particular patient will depend upon a variety of factors including the disorder being treated and the severity of the disorder; activity of the compound employed; the specific composition employed; the age, body weight, general health, sex and diet of the patient; the time of administration, route of administration and rate of excretion of the specific compound employed; the duration of the treatment; drugs used in combination or coincidental with the specific compound employed; and like factors well known in the medical arts. For example, it is well within the skill of the art to start at doses lower than required to achieve the desired therapeutic effect and to gradually increase the dosage until the desired effect is achieved.

### BRIEF DESCRIPTION OF THE DRAWINGS

Fig. 1 illustrates the influence of increasing sodium deoxycholate (g sodium deoxycholate/kg bypass cocktail) on weight gain in rainbow trout in all tanks;
Fig. 2 illustrates the influence of increasing sodium deoxycholate (g sodium deoxycholate/kg bypass cocktail) on weight gain in rainbow trout in extreme tanks;
Fig. 3 illustrates the percentage increase in brook trout weight in bST-supplemented bypass cocktail with increasing levels of sodium deoxycholate;
Fig. 4 illustrates fish weight gain of control and injected fish;
Fig. 5 illustrates the inhibition curve for freeze-dried ovalbumin of the Oralject^{™} formulation;
Fig. 6 illustrates the inhibition curve for red kidney beans of the Oralject^{™} formulation;
Fig. 7 illustrates the inhibition curve for soybeans of the Oralject^{™} formulation;
Fig. 8 illustrates the inhibition curve for faba beans of the Oralject^{™} formulation;
Fig. 9 illustrates the inhibition curve for EDTA of the Oralject^{™} formulation;
Fig. 10 illustrates the inhibition curve for wheat bran of the Oralject^{™} formulation;
Fig. 11 illustrates the inhibition curve for spray-dried ovalbumin of the Oralject^{™} formulation;
Fig. 12 illustrates the inhibition curve for combined ingredients of the Oralject^{™} formulation;
Fig. 13 illustrates the standard curve of HRP in the plasma of rainbow trout; and
Fig. 14 illustrates the effect of Oralject^{™} on HRP uptake.

### DETAILED DESCRIPTION OF THE INVENTION

The present invention relates to the administration of therapeutic proteins and polypeptides in oral dosage form. The invention provides increased absorption through the Gl tract and greatly improved bioavailability of the proteins/peptides as compared to that of the prior art formulations. The invention is useful both in human and veterinary nutrition, therapy and treatment. As used herein and in the appended claims, the term "polypeptide" encompasses proteins and peptides as well as polypeptides within its scope.

The compounds and compositions of the subject invention are useful for administering biologically or chemically active agents to any animals such as birds, fishes, mammals (such as primates and particularly humans), and insects. The system is particularly advantageous for delivering physiologically, biologically or chemically active agents which would otherwise be degraded or rendered less effective by conditions encountered before the active agent reaches its target zone (i.e. the area in which the active agent of the delivery composition is to be released) within the body of the animal to which they are administered. Particularly, the compounds and compositions of the present invention are useful for orally administering active agents, especially those which are not ordinarily orally deliverable.

The present invention is particularly useful for the administration of polypeptides, including proteins, such as, but not limited to, therapeutical agents, nutritional products, mucopolysaccharides, lipids, carbohydrates, steroids, hormones, growth hormone (GH), growth hormone releasing hormone (GHRH), epithelial growth factor, vascular endothelial growth and permeability factor (VEGPF), nerve growth factor, cytokines, interleukins, interferons, GMCSF, hormone-like product, neurological factor, neurotropic factor, neurotransmitter, neuromodulator, enzyme, antibody, peptide, proteic fragment, vaccine, adjuvant, an antigene, immune stimulating or inhibiting factor, heomatopoietic factor, anti-cancer product, anti-inflammatory agent, anti-parasitic compound, anti-microbial agent, nucleic acid fragment, plasmid DNA vector, cell proliferation inhibitor or activator, cell differentiating factor, blood coagulation factor, immunoglobulin, anti-angiogenic product, negative selective markers or "suicide" agent, toxic compound, anti-angiogenic agent, polypeptide, and anti-cancer agent nucleotides, and the like, and structurally similar bioactive equivalents thereof.

In accordance with one embodiment of the present invention, there is provided a composition for oral administration and intestinal delivery of a nutritional compound or a therapeutic polypeptide that can be formulated, but without limitation to products described herein, with deoxycholate and saponins in a ratio to provide a substantially increased absorption and systemic bioavailability of the peptide by the intestine of the host. The composition also comprises a pH neutralizing agent, such as but not limited to sodium carbonate and calcium carbonate, and at least one inhibitor of digestive enzymes, such as but not limited to egg albumin. This composition is preferably solid so as to be easy to manipulate in formulating oral composition forms. Neutralization of pH is intended to mean increasing the pH into the digestive tract to acid-base equilibrium compatible with most of known active biological products in nature or synthesized. The digestive tract's pH is increased to between about 6.5 and 9.

It is to be understood that the above list of drugs is for illustration purposes only and is not provided as an all inclusive list of all the drugs which may be beneficially formulated or reformulated using the oral delivery compositions of the present invention. Other physiologically-active compounds that can be encapsulated in the compositions of the present invention include biologically-active compounds, such as proteins, enzymes, anti-enzymes, peptides, catecholamines, anti-histamines, analgesics, and the like. For the purposes of the present invention "biological" is defined to mean any nutritionally or medically useful composition derived from a biological source and/or a synthetic pharmacological equivalent thereof such as insulin, heme, hemoglobin (bovine, human, or synthetic), and hormones; "enzyme" or "enzyme system" is defined to mean any protein or conjugated protein produced biologically or synthetically and which functions as a biocatalyst. Other medically useful compositions known to those skilled in the art, for example, globulin, one or more glycoproteins, such as erythropoeitin, may also be incorporated in the composition of the present invention.

The amount of therapeutic polypeptide will vary widely, depending on various factors such as the particular peptide to be delivered, the indication to be treated, the individual patient, and the like. The amount will be a therapeutically effective amount, that is, an amount that will provide a therapeutic effect, to be determined in accordance with well-established medical practice.

Another embodiment of the present invention is the use of enteric coatings, which are available for tablets and capsules. Enteric coatings will remain intact in the stomach but will rapidly dissolve once they arrive at the small intestine, thereafter releasing the drug at sites downstream in the intestine (e.g., the ileum and colon). Enteric coatings are well known in the art. Alternatively, a controlled release oral delivery vessel designed to release a drug after a predetermined period of time, and thus after the vessel has passed into the ileum or colon, can be used to deliver the formulation of the present invention. Such vessels include the CHRONSET^{™} delivery device (ALZA Corporation, Palo Alto, Calif.) and the Pulsincap^{™} delivery device (R.P. Scherer Co.).

The composition may further comprises an ion-pair forming reagent wherein the mole ratio of ion-pair forming reagent to drug is from about 2:1 to about 10:1. The ion-pair-forming reagent is added to increase the lipophilicity of the dissolved physiologically active agent or drug and thereby increase its membrane permeability. Increasing the drug's lipophilicity may also provide some protection of the drug from enzymatic deactivation as much of the peptide degradation that occurs *in vivo* does so in the aqueous environment of the gastrointestinal tract. Representative ion-pair forming reagents include sodium decanesulfonate, sodium lauryl sulfate, and sodium benzoate.

In one embodiment of the present invention is that the composition may optionally comprise from about 1 % to about 5% based on the total volume of the composition of an intestinal mucosal membrane transport enhancing agent, deoxycholate. Such agents facilitate the absorption of the therapeutic agent across the mucosal tissues in the intestinal mucosa and directly into the bloodstream of the subject. Also tissue transport enhancing agents suitable for use in the present compositions are selected from essential or volatile oils or from non-toxic, pharmaceutically acceptable organic and inorganic acids or salts and esters thereof. Essential or volatile oils which may be employed in the composition are selected from soybean oil, faba oil, rice oil, fish oil. The preferred essential oil is fish oil.

In another embodiment of the present invention, the composition may contain additional agents such as preservatives and antioxidants. Typical preservatives include sodium benzoate, sorbic acid, and the methyl and propyl esters of p-hydroxy-benzoic acid (parabens). Representative antioxidants include butylated hydroxy anisole, butylated hydroxy toluene, nordihydroguaiaretic acid, the gallates such as propyl gallate, hydroquinone, propenyl methyl guaethol and alkyl thiopropionates, or water soluble agents such as alkanolamines, alcohols, and propylene glycol. The most preferred antioxidant is Tenox^{™} GT1 (1:1 vitamin E-soybean oil), present in a concentration of between about 5% to about 25% based on the total volume of the droplet.

Oral absorption of recombinant human GH to carp is enhanced up to a 1000-fold when the delivered together with deoxycholate.

To prepare the pharmaceutical formulation of the present invention, the ingredients are dry blended together, after which the small amount of oil is added. These materials are mixed together until a homogeneous mixture of ingredient results. The resulting solid formulation can be pressed into tablets that can then be coated with a suitable enteric coating. Alternatively, the solid formulation can be placed into a capsule formed of gelatin or the like and coated with an enteric compound, or placed into a controlled release delivery device such as the CHRONSET^{™}. The solid formulation provides a mean for easily and conveniently fabricating a dosage form.

In one embodiment of the present invention, the composition comprises:

| | |
|---|---|
| Drug . | 5 mg/ml |
| Egg albumin | 10-20% |
| Sodium carbonate | 10-20% |
| Calcium carbonate | 10-20% |
| EDTA | 1-10% |
| Soybeans | 5-10% |
| Faba beans | 5-10% |
| Rice huul | 5-10% |
| Deoxycholate | 1-5% |
| Fish oil | 5-10% |
| Brewers yeast | 1-5% |

One embodiment of the invention is to provide a method for delivering hormones and pharmaceuticals to an animal or human host. Among the agricultural production field, the production of different species of fishes is importantly pointing out. The control of the reproduction physiology is of particular importance. The first indication of the invention for manipulating fish reproduction by feeding bioactive materials was provided by studies which employed mammalian and amphibian pituitaries. Thus, dietary replacement or supplementation with pituitary preparations has been observed to induce nuptial coloration in the bitterling *Acheilognathus inter-medium,* partially mature, and increase egg diameter, in the loach *Misgumus anguillicaudatus,* resulting in ovulation and a shortening in brood interval by 10-15 days in the swordtail *Xiphophorus helleri,* and increase egg size and induce precocious maturation in female lake trout *Salvelinus fontinalis.* Similarly, oral administration of salmon pituitary extract to goldfish *C. auratus,* induced ovulation and increased spermiation. The importance of these data relates to the accompanying elevations in plasma salmon gonadotropin (sGtH), testosterone and 17α-20fi-diphydroxy-4-pregnan-3-one, which provide a likely endocrine-based explanation for the observed effects of other pituitary preparations during earlier investigations (i.e., uptake of GtH).

Due to the problems inherent in using pituitary preparations and partially purified hormones, it would appear unlikely that such preparations will offer any major benefit with respect to the control of reproduction in cultured species using the oral route unless formulated in a composition of the present invention. A comparatively recent innovation in the control of maturation has been the application of gonadotropin-releasing hormone. Many of the analogue forms of GnRH are 50-100 times more effective at inducing ovulation than the natural forms, and include those which incorporate D amino acids and have terminal residues substituted with ethylamide. These manipulations have the effect of enhancing the resistance of the molecule to proteolysis. GnRHs stimulate the natural release of GtH, exhibit wide species potency, are relatively easy to manufacture and therefore, are economical. In addition, the peptides are stable over a wide range of temperature, and express non-varying potency. Importantly, the peptides are stable for an indefinite period provided they are stored in sterile conditions at temperatures below -20°C. As such GnRHAs provide excellent candidate molecules for use in the oral approach to controlling reproduction. Indeed, sufficient experimental evidence has accumulated, such that dietary delivery of GnRHAs, with or without dopamine agonist is now indicated as a method for controlling the final stages of maturation in fish. While more expensive than traditional methods (injection, implantation), dietary administration offers the advantage of being stress-free. This advance in reproductive biotechnology is particularly useful for species which are vulnerable to handling and/or, are too small for safe injection (i.e., ornamental species). In addition, chronic treatment with GnRHAs provides means to induce maturation precociously, which is considered advantageous during roe production, or for use with sex reversed broodstock.

Similar to the control of reproduction, studies using pituitaries as feed supplements also provide an early indication of the possibility of manipulating growth in teleosts using oral delivery techniques. Thus, it has been observed that the feeding of gruppies *Lebistes reticulatus,* with anterior pituitary powder resulted in significantly enhanced growth performance when compared against controls. Also, a 50% increase in length was observed for swordtails fed exclusively on dried anterior pituitary from birth, while other experiments observed a doubling in weight and tripling in length of lake trout fed anterior pituitary twice weekly. Treatment of cultured teleosts with growth hormone (GH) and related peptides offers a number of potential advantages, and several studies have confirmed that orally delivered GH not only enters the bloodstream, but accelerates growth rate in fish. Supplies of recombinant GH are presently stable and production could be increased many-fold with increased demand. Moreover, such recombinant proteins, when produced at the industrial level, are cost efficient and easily incorporated into commercial diets. While the structural integrity of the GH molecule may be of importance as a precursor to post-translational modified forms, methods of enhancing the molecules structural integrity or potency may provide benefit from an oral administration perspective. Description of growth-promoting fragments of the GH molecule may also provide products that express greater stability under lumenal degradation.

A particular embodiment of the present invention is to provide a composition and a use allowing the use of the oral route for vaccination that offers significant advantage in that it reduces labor costs, is time-saving, decreases the possibilities for cross-contamination with needles and does not involve inventory handling or require disposal of treatment waters.

The principal determinant of specific immunity at mucosal surfaces is secretory lgA (S-lgA) which is physiologically and functionally separate from the components of the circulatory immune system. S-lgA antibody responses may be induced locally by the application of suitable immunogens to a particular mucosal site. The bulk of mucosal S-lgA responses, however, are the results of immunity generated via the common mucosal immune system (CMIS), in which immunogens are taken up by specialized lympho-epithelial structures, collectively referred to as mucosa-associated lymphoid tissues (MALT). The best immunologic lymphoepithelial structures are the gut-associated lymphoid tissues (GALT), such as intestinal Peyer's patches. Other structurally and functionally similar lymphoid follicles occur at other mucosal surfaces, including those of the respiratory tract.

According to the present invention, a host can be immunized by oral administration of bacterial protein immunogens, preferably mixed with an adjuvant, such as cholera toxin (CT). Of course, as an adjuvant, the amount of cholera toxin used is non-toxic to the host.

The ability of a vaccine to protect against microbial colonization, as provided herein, means that the active component may protect against disease not only in the immunized host but, by eliminating carriage among immunized individuals, the pathogen and hence any disease it causes may be eliminated from the population as a whole.

Oral administration may also prevent sepsis resulting from administration of microbials, so that the vaccine can protect against both microbial colonization and sepsis (systemic infection).

For example, PspA is a preferred antigen for pneumococal infections. In published International patent application WO 92/14488, the entire content of which is incorporated herein by reference there are described DNA sequences for the PspA gene from S. pneumoniae Rx1, the production of a truncated form of PspA by genetic engineering and the demonstration that such truncated form of PspA confers protection in mice to challenge with live pneumococci.

From sequences of the PspA gene, it has been shown that PspA proteins are variable in size (roughly 70 kDa). The C-terminal 37% of the molecule is largely composed of the 20-amino acid repeats which form a binding site that permits PspA to attach to the phosphocholine residues of the pneumococcal lipoteichoic acids. The central region of PspA is rich in prolines and is suspected to be the portion of the molecule that passes through the cell wall. The sequence of the N-terminal 80% of the molecule is largely beta-helical and contains the region of PspA that can elicit antibodies that are protective against sepsis. Although PspA's are almost always at least slightly different from one another, there is enough cross-reactivity between them that antibodies or an immunological response to one PspA detect or is effective with respect to PspAs on all pneumococci. Moreover, immunization with one PspA can either protect against death or delay death with virtually all-different challenge strains. Accordingly, a mixture of a small number of PspA's could provide effective immunity against most pneumococci.

The immunoprotective truncated PspAs described in WO 92/14488 may be used in the present invention as the PspA fragments described above for oral administration.

Different vector systems for *in vitro* and *in vivo* expression of recombinant proteins are known; e.g., bacterial systems such as E. coli; and virus systems such as bacterial viruses, poxvirus (vaccinia, avipox virus, e.g., canarypox virus, fowlpox virus), baculovirus, herpes virus; yeast; and the like; and, these systems may be used for producing recombinant PspA using the coding gene thereof.

Immunogenicity may be improved if the antigen is co-administered with an adjuvant, commonly used as 0.001 % to 50% percent solution in phosphate buffered saline. Adjuvants enhance the immunogenicity of an antigen but are not necessarily immunogenic themselves. Adjuvants may act by retaining the antigen locally near the site of administration to produce a depot effect facilitating a slow, sustained release of antigen to cells of the immune system. Adjuvants may also attract cells of the immune system to an antigen depot and stimulate such cells to elicit immune responses.

Immunostimulatory agents or adjuvants have been used for many years to improve the host immune response to, for example, vaccines. Intrinsic adjuvants, such as lipopolysaccharides, normally are the components of the killed or attenuated bacteria used as vaccines. Extrinsic adjuvants are immunomodulators which are typically non-covalently linked to antigens and are formulated to enhance the host immune response. Aluminum hydroxide and aluminum phosphates (collectively commonly referred to as alum) are routinely used as adjuvants in human and veterinary vaccines. The efficacy of alum in increasing antibody responses to diphtheria and tetanus toxoids is well established and, more recently, a HBsAg vaccine has been adjuvanted with alum.

A wide range of extrinsic adjuvants can provoke potent immune responses to antigens. These include saponins complexed to membrane protein antigens (immune stimulating complexes), pluronic polymers with mineral oil, killed mycobacteria in mineral oil, Freund's complete adjuvant, bacterial products, such as muramyl dipeptide (MDP) and lipopolysaccharide (LPS), as well as lipid A, and liposomes. To efficiently induce humoral immune response (HIR) and cell-mediated immunity (CMI), immunogens are preferably emulsified in adjuvants.

Compositions of the invention, especially for oral administration may be conveniently provided as liquid preparations, e.g., isotonic aqueous solutions, suspensions, emulsions or viscous compositions which may be buffered to a selected pH. However, since delivery to the digestive tract is preferred, compositions of the invention may be in a "solid" form of pills, tablets, capsules, caplets and the like, including "solid" preparations which are time-released or which have a liquid filling, e.g., gelatin covered liquid, whereby the gelatin is dissolved in the stomach and/or small intestine for delivery to the gut and/or digestive system.

The composition of the invention may also contain pharmaceutically acceptable flavoring and/or coloring agents for rendering them more appealing. The viscous compositions may be in the form of gels, lotions, ointments, creams and the like and will typically contain a sufficient amount of a thickening agent so that the viscosity is from about 2500 to 6500 cps, although more viscous compositions, even up to 10,000 cps may be employed. Viscous compositions have a viscosity preferably of 2500 to 5000 cps, since above that range they become more difficult to administer. However, above that range, the compositions can approach solid or gelatin forms that are then easily administered as a swallowed pill for oral ingestion.

Liquid preparations are normally easier to prepare than gels and other viscous compositions, and solid compositions. Additionally, liquid compositions are somewhat more convenient to administer, especially to animals, children, particularly small children, and others who may have difficulty swallowing a pill, tablet, capsule or the like, or in multi-dose situations. Viscous compositions, on the other hand can be formulated within the appropriate viscosity range to provide longer contact periods with mucosa, such as the lining of the stomach or intestine.

Suitable nontoxic pharmaceutically acceptable carriers, and especially oral carriers, will be apparent to those skilled in the art of pharmaceutical and especially oral or peroral pharmaceutical formations. Obviously, the choice of suitable carriers will depend on the exact nature of the particular dosage form, e.g., liquid dosage form (e.g., whether the composition is to be formulated into a solution, a suspension, a gel or another liquid form, or a solid dosage form, or e.g., whether the composition is to be formulated into a pill, tablet, capsule, caplet, time release form or liquid-filled form).

Solutions, suspensions and gels, normally contain a major amount of water (preferably purified water) in addition to the antigen. Minor amounts of other ingredients such as pH adjusters (e.g., a base such as NaOH), emulsifiers or dispersing agents, buffering agents, preservatives, wetting agents, jelling agents, (e.g., methylcellulose), coloring and/or flavoring agents may also be present. The compositions can be isotonic, i.e., it can have the same osmotic pressure as blood and lacrimal fluid.

The desired isotonicity of the composition of this invention may be accomplished using sodium chloride, or other pharmaceutically acceptable agents such as dextrose, boric acid, sodium tartrate, propylene glycol or other inorganic or organic solutes. Sodium chloride is preferred particularly for buffers containing sodium ions.

Viscosity of the compositions may be maintained at the selected level using a pharmaceutically acceptable thickening agent. Methylcellulose is preferred because it is readily and economically available and is easy to work with. Other suitable thickening agents include, for example, xanthan gum, carboxymethyl cellulose, hydroxypropyl cellulose, carbomer, and the like. The preferred concentration of the thickener will depend upon the agent selected. The important point is to use an amount that will achieve the selected viscosity. Viscous compositions are normally prepared from solutions by the addition of such thickening agents.

A pharmaceutically acceptable preservative may be employed to increase the shelf life of the composition. Benzyl alcohol may be suitable, although a variety of preservatives including, for example, parabens, thimerosal, chlorobutanol, or benzalkonium chloride may also be employed. A suitable concentration of the preservative will be from 0.02% to 2% based on the total weight although there may be appreciable variation depending upon the agent selected.

Those skilled in the art will recognize that the components of the composition must be selected to be chemically inert with respect to microbial antigens. This will present no problem to those skilled in chemical and pharmaceutical principles, or problems can be readily avoided by reference to standard tests or by simple experiments (not involving undue experimentation), from this disclosure.

The immunologically effective compositions of this invention are prepared by mixing the ingredients following generally accepted procedures. For example the selected components may be simply mixed in a blender, or other standard device to produce a concentrated mixture which may then be adjusted to the final concentration and viscosity by the addition of water or thickening agent and possibly a buffer to control pH or an additional solute to control tonicity. Generally the pH may be from about 3 to 7.5. Compositions can be administered in dosages and by techniques well known to those skilled in the medical and veterinary arts taking into consideration such factors as the age, sex, weight, and condition of the particular patient or animal, and the composition form used for administration (e.g., solid vs. liquid). Dosages for humans or other mammals can be determined without undue experimentation by the skilled artisan.

When CT is used as an adjuvant for oral immunizations, specific IgA antibodies are induced in secretions. Strong circulatory immune responses can also be induced, with IgG and IgA antibodies in the serum, and IgG and IgA antibody-secreting cells in the spleen. The circulatory (or systemic) immune responses elicited by oral (peroral; intragastric) administration of microbial antigens along with CT are comparable with, or even stronger than, those induced by the administration of similar immunogens by the intragastric route. Accordingly, it appears that oral immunization is an effective route for stimulating common mucosal responses as well as circulatory antibody responses and can require less antigen than other immunization routes.

Most soluble or non-replicating antigens are poor immunogens, especially by the peroral route, probably because they are degraded by digestive enzymes and have little or no tropism for the GALT. A notable exception is CT, which is a potent mucosal immunogen, probably because of the G.sub.M1 ganglioside-binding property of this binding subunit, CTB, that enables it to be taken up by the M cells of Peyer's patches and passed to the underlying immunocompetent cells. In addition to being a good mucosal immunogen, CT is a powerful adjuvant. When administered in micrograms doses, CT greatly enhances immunogenicity of other soluble antigens co-administered with it.

In one embodiment, and in accordance with the present invention, there is provided a use of the composition in the manufacture of a medicament for treating a disease or disorder of a host by delivery of a therapeutic agent to the host after oral administration.

In another embodiment, cancer cells that may be treated in accordance with the present invention include malignant tumors. Malignant (including primary and metastatic) tumors which may be treated include, but are not limited to, those occurring in the adrenal glands; bladder, bone; breast; cervix; endocrine glands (including thyroid gland, the pituitary gland, and the pancreas) ; colon; rectum; heart; hematopoietic tissue; kidney; liver; lung; muscle; nervous system; brain; eye; oral cavity; pharynx; larynx; ovaries; prostate; skin (including melanoma); testicles; thymus, and uterus. It is to be understood, however, that the scope of the present invention is not to be limited to the treatment of any particular tumor.

In accordance with another preferred embodiment of the present invention, the agent which is capable of inhibiting, preventing, or destructing the cancer cells upon delivery of such agent is a negative selectable marker; i.e. a material which in combination with a chemotherapeutic or interaction agent inhibits, prevents or destroys the growth of the cancer tumor cells.

Thus, upon systemic delivery of negative selective marker, an interaction agent is administered to the animal or human host. The interaction agent interacts with the negative selective marker in order to prevent, inhibit, or destroy the growth of the cancer Negative selective markers which may be employed for example, but are not limited to, thymidine kinase, and cytosine deaminase.

The interaction agent is administered in an amount effective to inhibit, prevent, or destroy the growth of the cancer cells. For example, the interaction agent may be administered in an amount from about 5 mg to about 15 mg/kg of body weight, preferably about 10 mg/kg, depending on overall toxicity to a patient.

The present invention will be more readily understood by referring to the following examples which are given to illustrate the invention rather than to limit its scope.

### EXAMPLE I

### Growth enhancement of rainbow trout (Oncorhynchus mykiss) and Brook Trout (Salvelinus fontinalis): Feeding of recombinant bovine growth hormone using a novel delivery system

The aquaculture industry worldwide has undergone rapid expansion during the past 2 decades and currently represents the fastest growing agricultural segment. The sector has grown at an annual percentage rate of 10.9 since 1984, compared with 3.1 for terrestrial livestock meat production. The fastest growing livestock sector over the same period was chicken meat production with an APR of 5.3, followed by pig meat 3.4, mutton and lamb 1.4, and beef and veal 0.9. Aquaculture's contribution toward total world food fish landings has increased more than two fold since 1984 from 11.5% to 25.6% by weight in 1995. Projected increased demand for seafood products, coupled with decreased fisheries landings from wild stocks has, and will continue to contribute to the growth of the aquaculture industry.

The aquaculture industry, like other sectors of agriculture, faces many of the production challenges associated with traditional livestock production. Forty to fifty percent of the cost of salmonid production is attributed to feeding. Rations contain a high percentage of costly fish protein and salmonids require a relatively long feeding period to reach market weight. In fast growing fish, excessive fat deposition is a concern to both producers and consumers.

The goal of the food animal industry is to optimize production efficiency by minimizing the input of feed, labour, and capital investment while maximizing the yield of high quality protein. In the past, economically important parameters have been altered by genetic selection or nutritional modification. More recently, a variety of approaches have emerged involving endocrine system manipulation to influence growth and body composition of domestic animals. The ability of exogenous compounds to successfully alter the growth performance of domestic animals and offer potential savings in production costs, has prompted investigations into the use of these agents in fish.

The administration of growth hormone (GH) derived from various sources has provided evidence that this hormone plays a key role in stimulating somatic growth and reducing fat deposition in fish. Both native and recombinant piscine GH has been applied to several fish species, and are equipotents when injected into intact salmonids. As well, GH derived from mammalian and avian sources have been reported to be effective in altering growth performance of juvenile salmonids. Administration of bovine GH (bGH) to salmonids leads to a two to three-fold increase in growth rate, an increase in appetite and feed efficiency and reduction in adipose tissue. Exogenous GH is also effective in older (subadult) fish, and at low water temperatures when growth rate is depressed.

Oral application of GH is a practical method it has provided histochemical and biological evidence for a mechanism which transports intact proteins into the circulation of teleost fish following oral administration. It is now shown that orally administered horseradish peroxidase is transported to the circulation within 1 h.

It is reported the transfer of bGH into the circulatory system of yearling rainbow trout following introduction of the hormone into the lumen of the digestive tract. Similarly, it is demonstrated that recombinant salmon growth hormone (rsGH) administered orally, significantly elevated plasma rsGH concentrations. This same results show that weekly intragastric administration of rsGH resulted in a 50% increase in weight gain and fish length compared to control fish.

The above research supports that orally administered GH from various sources may influence growth performance in several teleost fish species, by being protected to eliminate gastric and intestinal digestion so that it remains intact and biologically active. This has given rise to several attempts to develop systems that will protect bioactive proteins (growth hormones, antigens, etc.) from the acidic environment of the stomach. Oral or rectal intubation of fish are effective methods to deliver bioactive proteins past the stomach, however, they are not feasible for commercial application. Attempts have been made to co-administer bioactive proteins along with detergents and antacids to reduce the acidic environment in the stomach. While these studies have demonstrated reduced protein degradation, the treatments used may affect the uptake of other important dietary factors. Another option is the use of pH-sensitive polymers which encapsulate and protect the peptides from acidic degradation in the stomach and permit release once in the small intestine.

It is clearly shown from the method of the present invention that compounds (hormones, vaccines, antibodies etc) are delivered orally past the stomach of monogastic animals (including humans) in order to bypass gastric digestion to the site of absorption in the small and/or large intestine(s). To date, a large proportion of this work has centered on developing encapsulation strategies using a range of formulations and forms of interest. These formulation and forms may simply modulate the release of a specific compound in a predetermined fashion, or may use specific physiological determinants (e.g. pH, temperature etc.) to trigger the delivery of the encapsulated material.

Complex polymer used in other forms of delivery systems is sometimes difficult to characterize. As well, the utilization of certain polymers that are not Generally Regarded as Safe (GRAS) makes regulatory approval of these systems a long and risky process. Furthermore, many polymer systems are relatively costly making large-scale utilization impractical.

The current experiment highlights a new strategy to permit oral delivery of a bioactive peptide (in this case bST). By feeding a bioactive compound of interest along with a cocktail of antinutritional factors to temporarily suppress digestive enzyme function and products that augment intestinal absorption (referred to as the 'bypass cocktail'), we have shown that we can effectively bypass the enzymatic process, and enhance intestinal uptake of the aforementioned compound to achieve a desired biological effect.

### Materials and Methods

### Bypass Cocktail Formulation

The formulation of the bypass cocktail is shown in Table 1. Unprocessed oilseed and pulse ingredients were obtained from local suppliers and mechanically dehulled. Fish meal, rice bran, brewers yeast, sodium carbonate, calcium carbonate and EDTA were all feed grade and purchased from local suppliers. Sodium deoxycholate and crude egg albumin were purchased from Sigma Chemical Co. (St Louis MO). The diet was mixed as indicated and ground using a 1 mm mesh.

**Table 1**

| **Bypass cocktail formulation** | |
|---|---|
| **Ingredient** | **Inclusion Rate (q/kg)** |
| Fish meal | 150 |
| Egg albumin | 100 |
| Soybeans (dehulled) | 100 |
| Kidney beans | 100 |
| Faba beans | 100 |
| Sodium carbonate | 100 |
| Calcium carbonate | 100 |
| Fish oil | 100 |
| Rice bran | 50 |
| EDTA | 50 |
| Brewers yeast | 45 |
| Sodium deoxycholate | 4-40¹ |

| | |
|---|---|
| ¹Concentration varied in experiments see Materials and Methods section | |

### Fish and Feeding

A series of experiments using two salmonid species was undertaken (Experiment 1: rainbow trout; Experiment 2: brook trout). These species were chosen as they represent both well studied experimental models as well as economically important cultured species.

For Experiment 1, rainbow trout (n=20; initial weight = 52 grams) were stocked into 6-60 liter cylindro-conic tanks in a closed water recirculation system 2 weeks prior to the start of the experiment. Water temperature was held at 15°C and photoperiod was set at 12hL:12hD cycle. In Experiment 2, brook trout (n= 400; initial weight 38 grams) were stocked into 8-800 liter cylindroconic tanks in a closed water recirculation system two weeks prior to the start of the experiment. Water temperature was 11°C for the duration of the experiment; fish were subjected to natural photoperiod (approx. 14hL:10hD). During both experiments water quality (ammonia, nitrite) was monitored weekly and oxygen concentrations measured daily. Fish were fed a commercial feed (Corey Feed Mills Ltd. Fredericton, NB) during the acclimation period and during the non-treatment periods.

### Experimental Manipulations

In Experiment 1, recombinant bovine somatotropin (rbST; Monsanto Co. St Louis MO) was included to provide fish with 20 µg/g fish. Three duplicate groups were fed varying levels of provide either 0 (control), 4 or 40 g sodium deoxycholate/kg bypass cocktail. In the second experiment, 4 duplicate treatment groups received food supplemented with 0, 1, 5 or 10 mg deoxycholate /kg bypass cocktail with 20 µg rbST/g fish.

In both Experiments 1 and 2, fish weight and feed consumption were monitored on a weekly basis. Fish were weighed and then fasted for 36 hours prior to feeding the bypass cocktail containing bST. Following feeding feed was withheld for an additional 12 hours. From this point, fish were fed twice daily to near satiation.

### Results

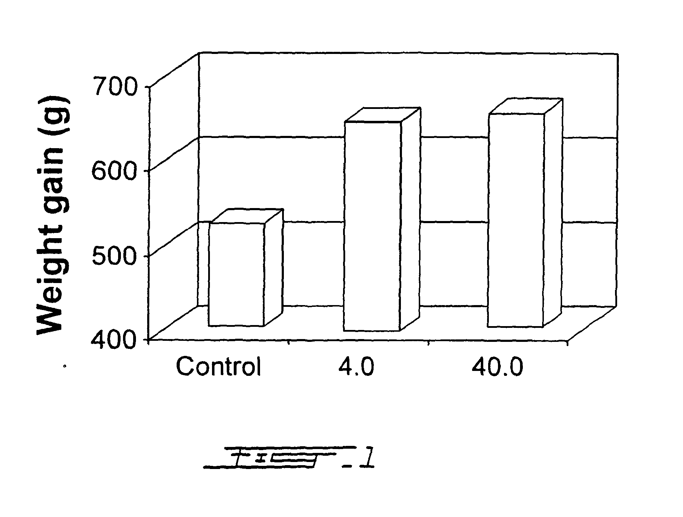

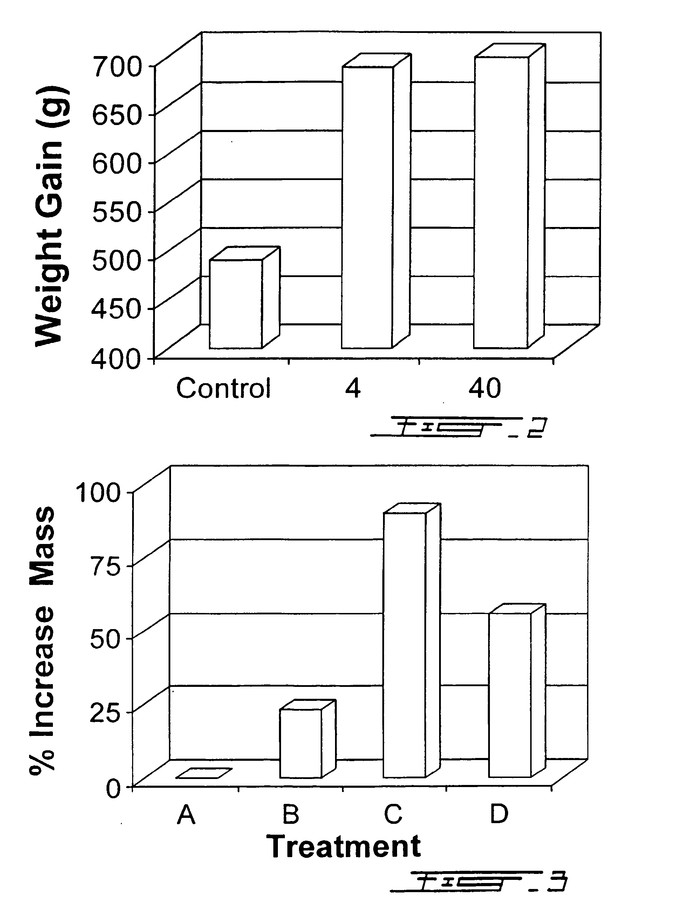

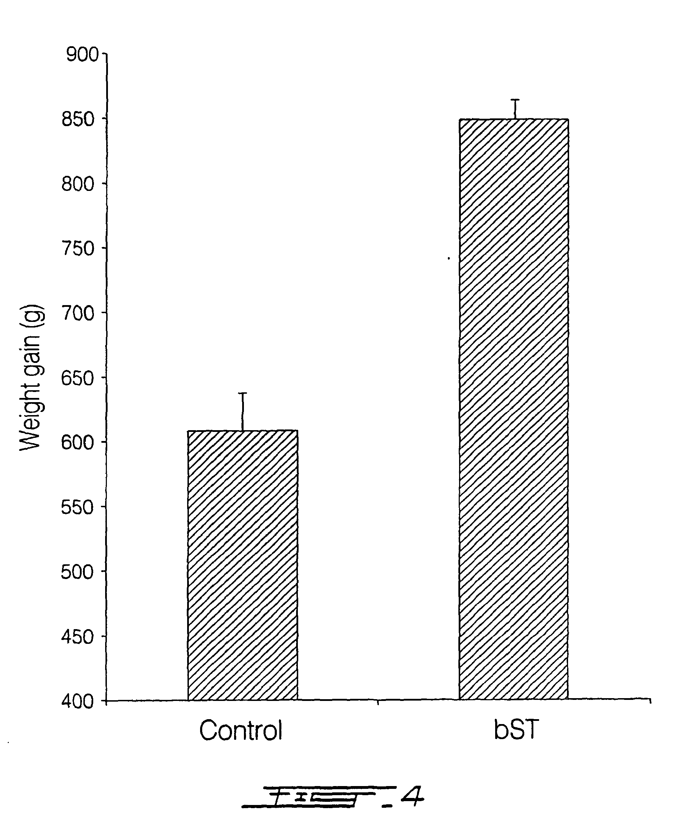

In both experiments, no treatment-associated mortalities were noted, suggesting no adverse health effects of the bST or the bypass cocktail on rainbow and brook trout. Fish fed bST in the bypass cocktail had significantly improved growth rates versus controls. In Experiment 1, treated fish averaged a 25% increase in growth rates; the fastest growing tanks grew over 40% larger than controls (Figs. 1 and 2). In Experiment 2, bST-treated groups showed improved growth rates versus controls, though those fed the bypass cocktail containing 5 g/kg deoxycholate showed the highest growth rates with a 90% increase in growth rates over controls (Fig. 3).

### EXAMPLE II (comparative)

### Growth enhancement of rainbow trout (Oncorhynchus mykiss) . Intraperitoneal injection of recombinant bovine growth hormone.

### Method

Intraperitoneal (IP) administration dose per fish weekly was 20 µg bST/g live body weight for 6 weeks.

### Results

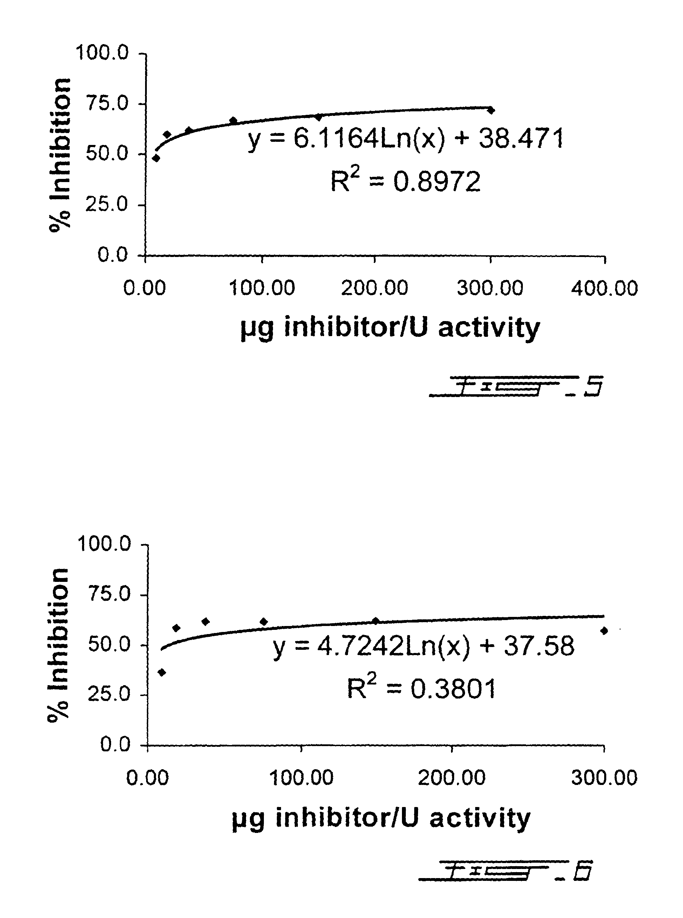

Fig. 4 illustrates that recombinant bST injected IP significantly induces increased body weight gain in rainbow trout.

### EXAMPLE III

### Assessment of proteolytic enzyme inhibitors present in feed ingredients

### Extract Enzymes protocol

### Materials

1. Centrifuge Sorvall model
2. Bench-top blender
3. Dissecting material (scissors)
4. Centrifuge bottle
5. Disposable cuvettes for spectrophotometer
6. Microcentrifuge tubes 1,5 ml
7. Spectrophotometer
8. Vortexer
9. Microplates reader from Biorad
10.50 mM Tris-HCl pH=7,5
11. Commassie blue staining solution
12. BSA (1 mg\ml) standard solution
13. TCA 20%
14. Rainbow trouts pancreatic and duodenal tissues
15.0,5% casein in 50 mM Tris-HCl pH=9
16.50 mM Tris-HCl+CaCl₂ 10 mM pH=7,5

### Enzyme extract

1. Rainbow trout were weighed and sacrificed.
2. Dissection was performed to remove the proximal small intestine from the fish.
3. After weighing, the tissues were homogenised in 50 mM Tris-HCl ph=7,5 (1:10 w\v).
4. Centrifuge at 16000Xg for 30 min at 4°C.
5. Keep the supernatant. Aliquot and store them at -20 C for further use.
6. Perform Commassie assay to measure the amount of protein present in the enzyme extract.

### Commassie blue stain protocol

1. Weigh 160 mg of BSA in 10 ml of 50 ml Tris-HCI pH=7,5.
2. Prepare a standard curve of BSA (0 µg/ml to 1600 µg/m)).
3. Add 4 µl in each well of either BSA, extract enzyme and dilute (1:1) extract enzyme in a 96 well plate.
4. Add 200 µl of Commassie blue.
5. Read at 655 nm with the microplate reader from Biorad.

### Enzymatic protocol:

The experiments are performed in duplicate.

### BLANK:

1. To 500 µl of 50 mM Tris-HCI+10 mM CaCl₂ pH=7,5 solution.
2. Add 500 _{I}II of TCA 20% (distilled water) solution.
3. Add 20 wl of enzyme extract
4. Add 500 _{I}II of casein 0,5% (50 mM Tris-HCI pH=9) solution.
5. Incubate 15 min at 4°C (ice). Centrifuge at 12000Xg for 5 min and read at 280 nm.

### TEST:

1. To 500 µl of 50 mM Tris-HCI+10 mM CaCl₂ pH=7,5 solution.
2. Add 20 µl enzyme extract.
3. Add 500 µl of casein 0,5% (50 mM Tris-HCI pH=9) solution.
4. Incubate at 0, 5,10,15 and 30 min at room temperature.
5. Stop the reaction by adding 500 µl de TCA 20%. Incubate 15 min at 4°C (ice). Centrifuge at 12000Xg for 5 min and read at 280 nm.

### Inhibitor extraction

1. Grind with the industrial grinder the food bought commercially in fine powder.
2. Weigh 250 mg of the powder and put it in 10 ml of 50 mM Tris-HCI pH=7,5 (final concentration should be 25 mg/ml).
3. With the hand tissues grinder, homogenise the solution.
4. Centrifuge 2000Xg for 10 min at room temperature*.
5. Keep the supernatant. It will be the inhibitor extract for the enzymatic protocol.

### Enzymatic protocol:

The experiments are performed in duplicate

### BLANK:

1. To 500 µl of 50 mM Tris-HCl+10 mM CaCl₂ pH=7,5 solution.
2. Add 500 µl of TCA 20% (distilled water) solution.
3. Add variable volumes of inhibitor extract or 50 mM Tris-HCI pH=7,5 solution.
4. Add 10 µl of enzyme extract.
5. Add 500 µl of casein 0,5% (50 mM Tris-HCl pH=9) solution.
6. Incubate 15 min at 4°C (ice). Centrifuge at 12000Xg for 5 min and read at 280 nm.

### CONTROL:

1. To 500 gl of 50 mM Tris-HCl+10 mM CaCl₂ pH=7,5 solution.
2. Add variables volumes of 50 mM Tris-HCl pH=7,5 solution.
3. Add 10 µl enzyme extract.
4. Incubate 60 min at room temperature.
5. Add 500 _{I}II of casein 0,5% (50 mM Tris-HCl pH=9) solution.
6. Incubate at 30 min at room temperature.
7. Stop the reaction by adding 500 µl de TCA 20%. Incubate 15 min at 4°C (ice). Centrifuge at 12000Xg for 5 min and read at 280 nm.

### TEST:

1. To 500 µl of 50 mM Tris-HCl+10 mM CaCl₂ pH=7,5 solution.
2. Add variables volumes of inhibitor extract.
3. Add 10 µl enzyme extract.
4. Incubate 60 min at room temperature.
5. Add 500 µl of casein 0,5% (50 mM Tris-HCl pH=9) solution.
6. Incubate at 30 min at room temperature.
7. Stop the reaction by adding 500 µl de TCA 20%. Incubate 15 min at 4°C (ice).
8. Centrifuge at 12000Xg for 5 min and read at 280 nm.

### Results

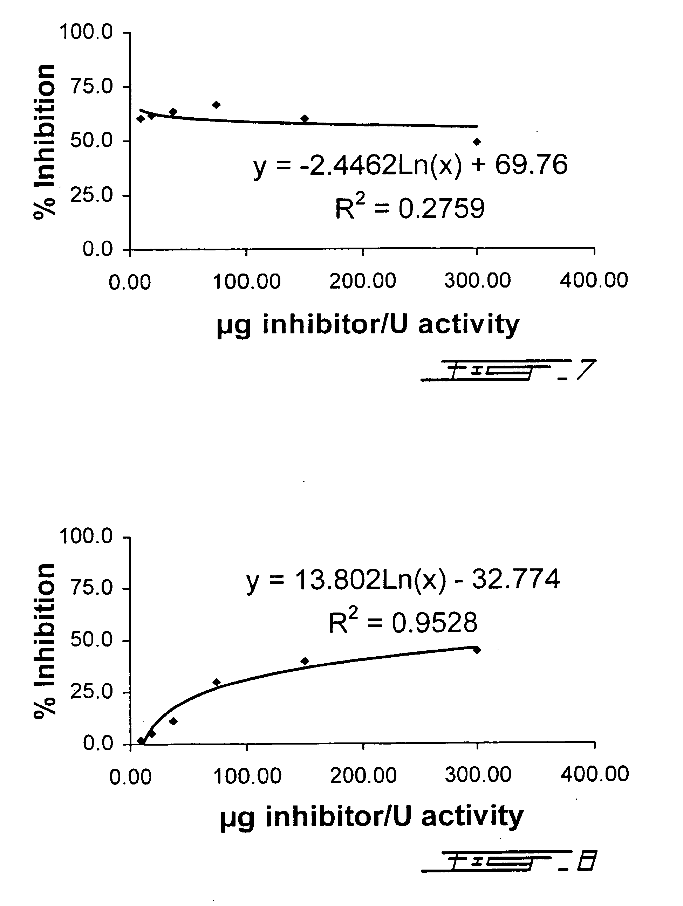

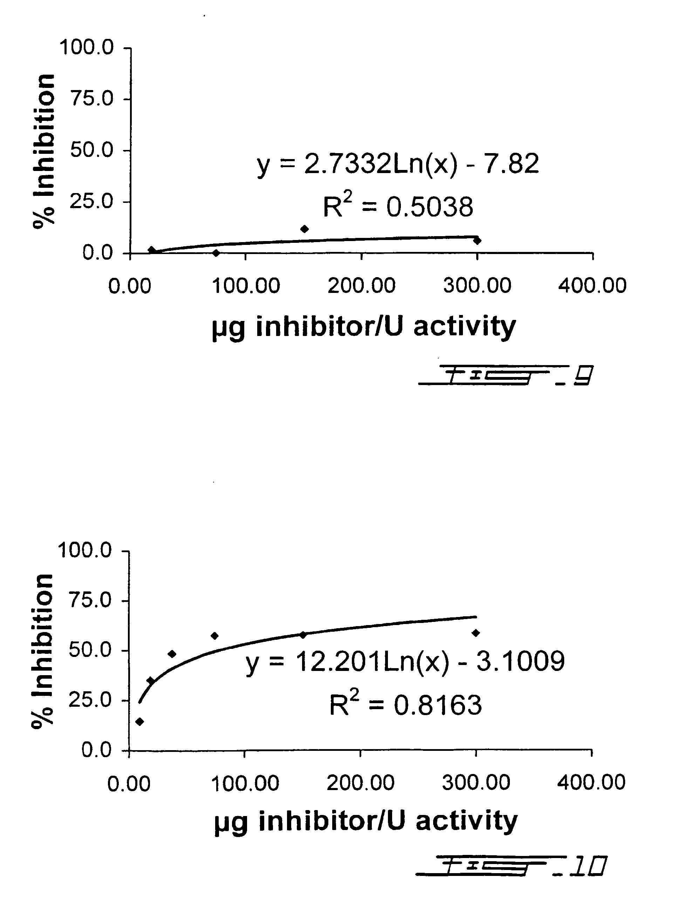

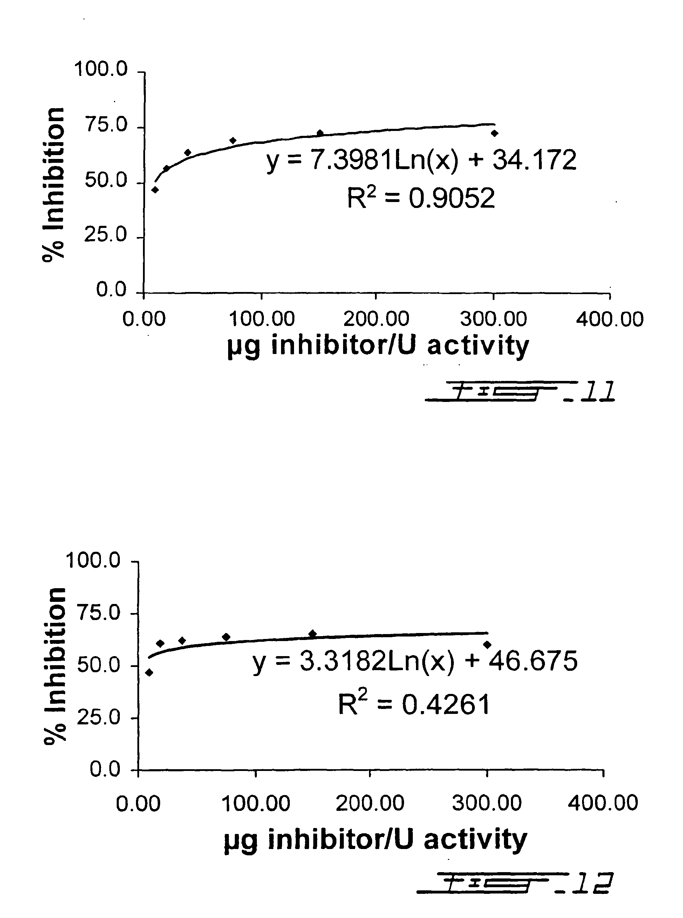

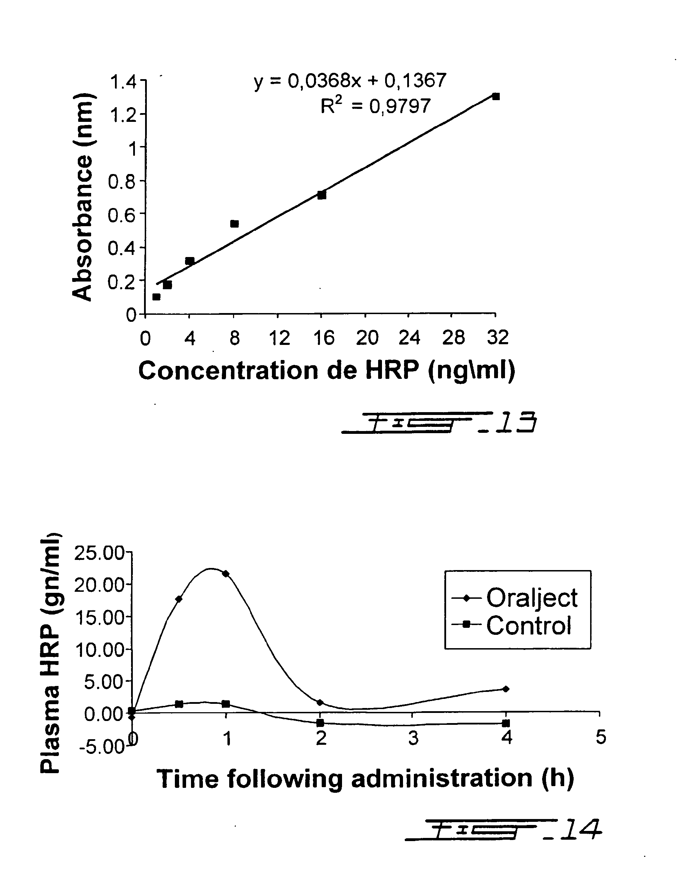

Figs. 5-12 demonstrate the effects of individual protease inhibitor components of the Oralject^{™} cocktail on *in vitro* proteolytic inhibition, as well as the overall inhibition of the Oralject^{™} cocktail. These data are presented as the degree of proteolytic enzyme inhibition versus increasing level of inhibitor inclusion. The data reveal that the individual components (lyophylized ovalbumin, red kidney beans, soybeans, faba beans, EDTA, wheat bran, spray-dried ovalbumin, Figs 5 - 12 respectively) of the Oralject^{™} cocktail affect to differing degrees the inhibition of *in vitro* proteolytic enzyme activity. Furthermore the overall cocktail is effective in inducing overall proteolytic enzyme inhibition. Finally, using the curves generated in Figs. 5 to 12, the point of maximal inhibition as well as the concentration of inhibitor providing 50% of the maximal inhibition were extrapolated.

### EXAMPLE IV

### Enzyme assay for the quantification of horseradish peroxidase in the blood of rainbow trout

### Material

1. 96 well plates (Immulon^{™} II from VWR)
2. Microplate reader from Biorad^{™}
3. Microcentrifuge tubes of 1,5 ml
4. Centrifuge tubes of 15 ml or 50 ml
5. TMB tablets
6. Horseradish peroxidase type 1 (Sigma)
7. Anti-Horseradish peroxidase from goat IgG (ICN)
8. 0,1 M carbonate-bicarbonate pH=9,6 buffer
9. 0,1 M phosphate-citrate pH=5 buffer
10. PBS 1 X+BSA 1%+0,5% Tween 20 buffer
11. PBS 1X pH=7,4 buffer
12. Hydrogen peroxide 30%
13. Saran wrap
14. Incubator at 37°C
15. Distilled water
16. Rainbow trout (plasma)

### Method

### Coat plate with antigen

1. Dispensed 200 _{I}II of the anti-HRP from goat IgG dilute 1:1000 solution (in 0,1 M carbonate-bicarbonate pH=9,6 buffer) into each well of a 96 wells plate.
2. Wrap coated plate in saran^{™} wrap to seal and incubate overnight at 4°C or 2 hours at 37°C.
3. Rinse coated plate 3 times with PBS 1 X pH=7,4. Each time, flick the phosphate-buffered saline into the sink and rinse 3 more times with distilled water.
**4. The plates were shaken dry and stored at 4°C until use.**

### Block residual binding capacity of plate

1. Fill each well with 200 µl of PBS 1X+BSA 1%+0,5% Tween 20 buffer.
2. Incubate 30 minutes at room temperature.
3. Add 100 µl of the sample containing HRP dilute 1:10 in some wells.
4. Add 100 µl of standard curve plasma in the others wells.
5. Wrap the plate in the saran wrap and incubate 1 hour at 37°C.
6. Rinse 3 times with PBS 1X+BSA 1%+0,5% Tween 20 buffer.

### Standard curve method

1. Dilute the plasma of the rainbow trout 1:10 with PBS 1X pH=7,4.
2. Add HRP to obtain a final concentration of 0,5 to 8 ng/ml.

### Enzyme assay

1. Add 200 µl of TMB (in 50 mM citrate-phosphate pH=5 buffer+ 30% of hydrogen peroxide) in each well.
2. Wait 30 minutes and add 50 µl of 1M sulfuric acid to fix the coloration.
3. Read at 415 nm with the Microplate reader from Biorad^{™}.

### Results

An ELISA was developed for horseradish peroxidase (HRP), permitting its use as a tracer for plasma uptake studies following oral administration. This method has provided an extremely sensitive method to document HRP uptake with lower detection limit of approximately 2.5 ng HRP/ ml plasma and a linear portion up to 8 ng/ ml (Fig. 13).

Using this method to follow HRP uptake, a fish meal-based control matrix, and the Oralject^{™} cocktail containing HRP (2.5 ng/g) was force fed to rainbow trout and blood samples taken at selected times following administration. As illustrated in Fig. 14, plasma uptake of orally delivered HRP in the Oralject^{™} formulation was significantly higher than that of the fish meal control. Furthermore, the circulating concentrations of HRP were detected for a period of 6h following administration.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

## Claims

1. A composition for oral administration to a human or an animal for intestinal delivery of a physiologically active agent, said composition comprising:
a) at least one neutralizing agent suitable for increasing gastric pH to between about 6.5 to 9 in said animal digestive system to prevent denaturation of said physiologically active agent;
b) at least one inhibitor of digestive enzymes to prevent enzymatic digestion of said physiologically active agent; and
c) at least one uptake-increasing agent which increases intestinal absorption of said physiologically active agent.

2. The composition as claimed in claim 1, wherein said neutralizing agent is at concentration between 1% to 60% w/w, said inhibitor is at concentration between 1% to 50% w/w, and said uptake increasing agent is at concentration between 0.1% to 50% w/w.

3. The composition as claimed in claim 1, which further comprises a physiologically active agent selected from the group consisting of therapeutical agents, nutritional products, mucopolysaccharides, lipids, carbohydrates, steroids, hormones, growth hormone (GH), growth hormone releasing hormone (GHRH), epithelial growth factor, vascular endothelial growth and permeability factor (VEGPF), nerve growth factor, cytokines, interleukins, interferons, GMCSF, hormone-like product, neurological factor, neurotropic factor, neurotransmitter, neuromodulator, enzyme, antibody, peptide, proteic fragment, vaccine, adjuvant, an antigcnc antigen, immune stimulating or inhibiting factor, hematopoietic factor, anti-cancer product, anti-inflammatory agent, anti-parasitic compound, anti-microbial agent, nucleic acid fragment, plasmid DNA vector, cell proliferation inhibitor or activator, cell differentiating factor, blood coagulation factor, immunoglobulin, anti-angiogenic product, negative selective markers or "suicide"agent, toxic compound, anti-angiogenic agent, polypeptide, anticancer agent, acid production drugs, and histamine H2-receptor antagonist.

4. The composition as claimed in claim 1, wherein said neutralizing agent is in amount sufficient to neutralize acidic degradation in said animal digestive system and allow delivery of said physiologically active agent to said animal intestine.

5. The composition as claimed in claim 1, wherein said neutralizing agent is selected from the group consisting of anti-acids, sodium bicarbonate, sodium carbonate, sodium citrate, sodium hydrogencarbonate, calcium phosphate, calcium carbonate, magnesium salts, magnesium carbonate, magnesium trisilicate, magnesium hydroxide, magnesium phosphate, magnesium oxide, bismuth subcarbonate, and combinations thereof.

6. The composition as claimed in claim 5, wherein said neutralizing agent is at least one of sodium carbonate at a concentration of 10% to 20% w/w, and calcium carbonate at concentration of 10% to 20% w/w of the composition.

7. The composition as claimed in claim 1, wherein said inhibitor is in an amount sufficient to inhibit degradation of said physiologically active agent by digestive enzymes in said animal digestive system and allow delivery of said physiologically active agent to said animal intestine.

8. The composition as claimed in claim 1, wherein said inhibitor of digestive enzymes is selected from the group consisting of anti-protease, egg albumin, plant-derived inhibitors from oilseed, soybean, kidney bean, faba bean, rice bran, wheat bran, ethylenediamine tetraacetate, alpha-1-antitrypsin, albumin, ovalbumin, and proteosomes.

9. The composition as claimed in claim 8, wherein said inhibitor comprises at least one of a pepsin inhibitor and an enteropeptidase inhibitor.

10. The composition as claimed in claim 8, wherein said inhibitor is albumin at a concentration between 10% to 20% w/w.

11. The composition as claimed in claim 1, wherein said uptake increasing agent is selected from the group consisting of bile salt, saponin, deoxycholate, sodium salicylate, sodium lauryl sulphate, oleic acid, linoleic acid, monoolein, lecithin, lysolecithin, polyoxyethylene sorbitan ester, p-t-octylphenoxypolyoxyethylene, N-lauryl-p-D-maltopyranoside, idodecylazacycloheptane-2-azone, and phospholipid.

12. The composition as claimed in claim 11, wherein said uptake-increasing agent is deoxycholate at a concentration between 1% to 5%.

13. The composition as claimed in claim 1, further comprising at least one additional ingredient selected from the group consisting of ethylenediamine tetraacetate, preservative, actioxidant, colorant, binder, tracer, sweetener, surfactant, unmoulding agent, flavouring agent, meal, bean, yeast, brewer yeast, mineral oil, vegetable oil, animal oil, lubricant, ointment, and combinations thereof.

14. The composition as claimed in claim 3, wherein said physiologically active agent when delivered in said human or animal intestine is absorbed by said intestine for systemic delivery.

15. The composition as claimed in claim 3, wherein said physiologically active agent when delivered in said human or animal intestine has an effective physiological effect on intestinal wall.

16. The composition as claimed in claim 3, wherein said physiologically active agent when delivered in said human or animal intestine has a physiological effect on the content of the intestine.

17. The composition as claimed in claim 1, wherein said animal is a bird, a mammal, an insect, or a fish.

18. The composition as claimed in claim 3, wherein said physiologically active agent is capable of inducing an immune response in said human or animal against mucosal infectious diseases.

19. A medicament comprising the composition as claimed in any one of preceding claims.

20. A medicament comprising a sufficient amount of the composition as claimed in claim 3, 14, 15 or 16.

21. The medicament as claimed in claim 20, for systemic delivery of a physiologically active agent to human or an animal.

22. The medicament as claimed in claim 20, wherein said physiologically active agent is an antimicrobial agent.

23. The medicament as claimed in any one of claims 19 to 22, for treating an intestinal microbial infection in a human or an animal.

24. The medicament as claimed in claim 23, wherein said microbial infections are caused by microorganisms selected from the group consisting of bacteriae, mushrooms, yeasts, viruses, *Staphylococci, Streptococci, Micrococci, Peptococci, Peptostreptococci, Enterococci, Bacillus, Clostridium, Lactobacillus, Listera, Erysipelothrix, Propionibacterium, Eubacterium, Corynobacterium, Mycoplasma, Ureaplasma, Streptomyces, Haemophilus, Nesseria, Eikenellus, Moraxellus, Actinobacillus, Pasteurella, Bacteroides, Fusobacteria, Prevotella, Porphyromonas, Veillonella, Treponema, Mitsuokella, Capnocytophaga, Campylobacter, Klebsiella, Chlamydia,* and Coliforms.

25. The medicament as claimed in claim 22, wherein said antimicrobial agent is selected from the group consisting of antibiotics, bacteriocins, lantibiotics, probiotics, antifungics, antimycotics, antiparasitics, aminoglycosides, vancomycin, rifampin, lincomycin, chloramphenicol, and the fluoroquinol, penicillin, beta-lactams, amoxicillin, ampicillin, azlocillin, carbenicillin,mezlocillin, nafcillin, oxacillin, piperacillin, ticarcillin, ceftazidime, ceftizoxime, ceftriaxone, cefuroxime, cephalexin, cephalothin, imipenen, aztreonam, gentamicin, netilmicin, tobramycin, tetracyclines, sulfonamides, macrolides, erythromicin, clarithromcin, azithromycin, polymyxin B, and clindamycin antibiotic.

26. A medicament comprising a physiologically effective amount of a composition as claimed in claim 3, for enhancing intestinal uptake of the physiologically active agent in humans or animals.

27. Use of a composition according to claim 1 to 18 in the manufacture of a medicament for enhancing intestinal uptake of the physiologically active agent in humans or animals.

## Patentansprüche

1. Zusammensetzung zur oralen Verabreichung an einem Menschen oder einem Tier zur intestinalen Abgabe eines physiologisch aktiven Agens, wobei diese Zusammensetzung enthält:
a) wenigstens ein neutralisierendes Agens, das zur Anhebung des im Magen herrschenden pH-Werts auf Werte zwischen ungefähr 6,5 bis 9 in dem Verdauungssystem des besagten Tiers geeignet ist, um die Denaturierung des besagten physiologisch aktiven Agens zu verhindern;
b) wenigstens einen Inhibitor von Verdauungsenzymen zur Verhinderung der enzymatischen Verdauung des besagten physiologisch aktiven Agens; und
c) wenigstens eines die Aufnahme verstärkenden Agens, das die intestinale Absorption des besagten physiologisch aktiven Agens erhöht.

2. Zusammensetzung wie in Anspruch 1 beansprucht, wobei das neutralisierende Agens in einer Konzentration zwischen 1 bis 60 Gew.-%, der besagte Inhibitor in einer Konzentration zwischen 1 bis 50 Gew.-%, und das die Aufnahme fördernde Agens in einer Konzentration zwischen 0,1 bis 50 Gew.-% vorhanden ist.

3. Zusammensetzung wie in Anspruch 1 beansprucht, die weiterhin enthält ein physiologisch aktives Agens ausgewählt aus der Gruppe bestehend aus therapeutischen Agenzien, Ernährungsprodukten, Mucopolysaccariden, Lipiden, Kohlenhydraten, Steroiden, Hormonen, Wachstumshormon (GH), Wachstumshormon abgebendes Hormon (growth hormone releasing hormone GHRH), epithelialem Wachstumsfaktor, vaskulärem endothelialem Wachstums- und Permeabilitätsfaktor (vascular endothelial growth and permeability factor VEGPF), Nervenwachstumsfaktor (nerve growth factor), Cytokinen, Interleukinen, Interferonen, GMCSF, hormonähnlichem Produkt, neurologischem Faktor, neurotropem Faktor, Neurotransmitter, Neuromodulator, Enzym, Antikörper, Peptid, Protein-artiges Fragment, Vaccin, Adjuvans, ein Antigen, Immunstimulierender oder -inhibierender Faktor, hämatopoetischer Faktor, Anti-Krebsprodukt, antiinflammatorisches Agens, antiparasitäre Verbindung, antimikrobielles Agens, Nukleinsäurefragment, Plasmid-DNA-Vektor, Zellproliferationsinhibitor oder -aktivator, Zelldifferenzierungsfaktor, Blutgerinnungsfaktor, Immunoglobulin, antiangionetisches Produkt, negative selektive Marker oder "Suizid"-Agens, toxische Verbindung, Anti-Angionese-Agens, Polypeptid, Antikrebsagens, Säureproduktions-Medikamente und Histamin H2-Rezeptor Antagonist.

4. Zusammensetzung wie in Anspruch 1 beansprucht, wobei das besagte neutralisierende Agens in einer Menge vorhanden ist, die ausreichend ist zur Neutralisierung des Abbaus durch Säuren in dem Verdauungssystem des besagten Tieres, um die Abgabe des besagten physiologischen Agens zu dem besagten Tierdarm zu gestatten.

5. Zusammensetzung wie in Anspruch 1 beansprucht, wobei das besagte neutralisierende Agens ausgewählt ist aus der Gruppe bestehend aus Anti-Säuren, Natriumbicarbonat, Natriumcarbonat, Natriumcitrat, Natriumhydrogencarbonat, Calciumphosphat, Calciumcarbonat, Magnesiumsalze, Magnesiumcarbonat, Magnesiumtrisilikat, Magnesiumhydroxid, Magnesiumphosphat, Magnesiumoxid, Wismutsubcarbonat und Kombinationen davon.

6. Zusammensetzung wie in Anspruch 5 beansprucht, wobei das neutralisierende Agens zumindest eines aus der Gruppe bestehend aus Natriumcarbonat in einer Konzentration von 10 bis 20 Gew.-% und Calciumcarbonat in einer Konzentration von 10 bis 20 Gew.-% bezogen auf die Zusammensetzung ist.

7. Zusammensetzung wie in Anspruch 1 beansprucht, wobei der besagte Inhibitor in einer Menge vorhanden ist, die ausreichend ist zur Verhinderung des Abbaus des besagten physiologisch aktiven Agens durch Verdauungsenzyme in dem Verdauungssystem des besagten Tieres, und um die Abgabe des besagten physiologisch aktiven Agens zu dem Darm des besagten Tiers zu gestatten.

8. Zusammensetzung wie in Anspruch 1 beansprucht, wobei der besagte Inhibitor der Verdauungsenzyme ausgewählt ist aus der Gruppe bestehend aus Antiprotease, Eialbumin, Inhibitoren auf Pflanzenbasis aus Ölsaat, Sojabohne, Kidneybohne, Saubohne, Reiskleie, Weizenkleie, Ethylendiamintetraacetat, alpha-1-Antitrypsin, Albumin, Ovalbumin, und Proteosomen.

9. Zusammensetzung wie in Anspruch 8 beansprucht, wobei der besagte Inhibitor wenigstens einen aus der Gruppe Pepsin-Inhibitor und Enteropeptidase-Inhibitor enthält.

10. Zusammensetzung wie in Anspruch 8 beansprucht, wobei der besagte Inhibitor Albumin in einer Konzentration zwischen 10 bis 20 Gew.-% ist.

11. Zusammensetzung wie in Anspruch 1 beansprucht, wobei das besagte, die Aufnahme verstärkende Agens ausgewählt ist aus der Gruppe bestehend aus Gallensalz, Saponin, Desoxycholat, Natriumsalicylat, Natriumlaurylsulfat, Ölsäure, Linolsäure, Monoolein, Lecithin, Lysolecithin, Polyoxyethylen-Sorbitanestern, p-t-Octylphenoxypolyoxyethylen, N-Lauryl-p-D-maltopyranosid, 1-Dodecylazacyloheptan-2-azon, und Phospholipid.

12. Zusammensetzung wie in Anspruch 11 beansprucht, wobei das besagte, die Aufnahme verstärkende Agens Desoxycholat in einer Konzentration von 1 bis 5 % ist.

13. Zusammensetzung wie in Anspruch 1 beansprucht, weiterhin enthaltend wenigstens einen zusätzlichen Bestandteil ausgewählt aus der Gruppe bestehend aus Ethylendiamintetraacetat, Konservierungsmittel, Antioxidans, Farbstoff, Binder, Tracer, Süßungsmittel, oberflächenaktives Mittel, Trennmittel, Aromastoff, Mehl, Bohne, Hefe, Brauereihefe, Mineralöl, Pflanzenöl, tierisches Öl, Gleitmittel, Salbe, und Kombinationen davon.

14. Zusammensetzung wie in Anspruch 3 beansprucht, wobei das besagte physiologisch aktive Agens bei der Abgabe in dem besagten Mensch- oder Tierdarm von dem besagten Darm zur systemischen Abgabe absorbiert wird.

15. Zusammensetzung wie in Anspruch 3 beansprucht, wobei das besagte physiologisch aktive Agens bei der Abgabe in dem besagten Menschen- oder Tierdarm einen effektiven physiologischen Effekt auf die Darmwand ausübt.

16. Zusammensetzung wie in Anspruch 3 beansprucht, wobei das besagte physiologisch aktive Agens bei der Abgabe in dem besagten Menschen- oder Tierdarm einen physiologischen Effekt auf den Inhalt des Darms hat.

17. Zusammensetzung wie in Anspruch 1 beansprucht, wobei das besagte Tier ein Vogel, ein Säugetier, ein Insekt, oder ein Fisch ist.

18. Zusammensetzung wie in Anspruch 3 beansprucht, wobei das besagte physiologisch aktive Agens in der Lage ist, eine Immunantwort in dem besagten Menschen oder Tier gegen Schleimhaut-Infektionskrankheiten auszulösen.

19. Medikament enthaltend die Zusammensetzung wie in irgendeinem der vorhergehenden Ansprüche beansprucht.

20. Medikament enthaltend eine ausreichende Menge der Zusammensetzung wie in Anspruch 3, 14, 1 oder 16 beansprucht.

21. Medikament wie in Anspruch 20 beansprucht, zur systemischen Abgabe eines physiologisch aktiven Agens an einen Menschen oder ein Tier.

22. Medikament wie in Anspruch 20 beansprucht, wobei das besagte physiologisch aktive Agens ein antimikrobielles Agens ist.

23. Medikament wie in einem der Ansprüche 19 bis 22 beansprucht, zur Behandlung einer mikrobiellen Darminfektion in einem Menschen oder einem Tier.

24. Medikament wie in Anspruch 23 beansprucht, wobei die besagten mikrobiellen Infektionen hervorgerufen werden durch Mikroorganismen ausgewählt aus der Gruppe bestehend aus Bakterien, Pilzen, Hefen, Viren, *Staphylokokken, Streptokokken, Mikrokokken, Peptokokken, Peptostreptokokken, Enterokokken, Bazillus, Clostridium, Lactobazillus, Listeria, Erysipelothrix, Propionibakterium, Eubacterium, Corynobakterium, Mykoplasma, Ureaplasma, Steptromyces, Hämophilus, Nesseria, Eikenellus, Moraxellus, Actinobazillus, Pasteurella, Bakteroides, Fusobacteria, Prevotella, Porphyromonas, Veillonella, Treponema, Mitsuokella, Capnocytophaga, Camphylobacter, Klebsiella, Chlamydia, und Coliforme.*

25. Medikament wie in Anspruch 22 beansprucht, wobei das besagte antimikrobielle Agens ausgewählt ist aus der Gruppe bestehend aus Antibiotika, Bakteriozinen, Lantibiotika, Probiotika, Antipilzmittel, Antimykotika, antiparasitären Mitteln, Aminoglykosiden, Vancomycin, Rifampin, Lincomycin, Chloramphenicol und den Antibiotika Fluorquinol, Penicillin, beta-Lactame, Amoxicillin, Ampicillin, Azlocillin, Carbenicillin, Mezlocillin, Nafcillin, Oxacillin, Piperacillin, Ticarcillin, Ceftazidim, Ceftizoxim, Ceftriaxon, Cefuroxim, Cephalexin, Cephalothin, Imipenen, Aztreonam, Gentamicin, Netilmicin, Tobramycin, Tetracycline, Sulfonamide, Macrolide, Erythromycin, Clarithromcin, Azitrhomycin, Polymyxin B, und Clindamycin-Antibiotika.

26. Medikament enthaltend eine physiologisch wirksame Menge einer Zusammensetzung wie in Anspruch 3 beansprucht, zur Verbesserung der Darmaufnahme des physiologisch aktiven Agens in Menschen oder Tieren.

27. Verwendung einer Zusammensetzung nach Anspruch 1 bis 18 zur Herstellung eines Medikaments zur Verbesserung der Darmaufnahme des physiologisch aktiven Agens in Menschen oder Tieren.

## Revendications

1. Une composition pour administration par voie orale à un humain ou un animal pour la distribution dans l'intestin d'un agent physiologiquement actif, ladite composition comprenant:
a) au moins un agent de neutralisation permettant l'augmentation du pH gastrique entre environ 6,5 à 9,0 dans le système digestif dudit animal pour empêcher la dénaturation dudit agent physiologiquement actif;
b) au moins un inhibiteur des enzymes de digestion pour empêcher la digestion enzymatique dudit agent physiologiquement actif; et
c) au moins un agent augmentant la capture qui augmente l'absorption intestinale dudit agent physiologiquement actif.

2. La composition selon la revendication 1, **caractérisée en ce que** ledit agent de neutralisation est à une concentration entre 1 % et 60% m/m, ledit inhibiteur est à une concentration entre 1% et 50% m/m, et ledit agent augmentant la capture est à une concentration entre 0,1 % et 50% m/m.

3. La composition selon la revendication 1, laquelle comprend en plus un agent physiologiquement actif choisi parmi le groupe comprenant des agents thérapeutiques, des produits de nutrition, des mucopolysaccharides, des lipides, des glucides, des stéroïdes, des hormones, l'hormone de croissance (GH), le facteur de libération de l'hormone de croissance (GHRH), le facteur de croissance épithéliale, le facteur de croissance vasculaire endothéliale et de perméabilité (VEGPF), le facteur de croissance du tissu nerveux, des cytokines, des interleukines, des interférons, GMCSF, un produit à comportement hormonal, un facteur neurologique, un facteur neurotrope, un neurotransmetteur, un neuromodulateur, un enzyme, un anticorps, un peptide, un fragment protéique, un vaccin, un adjuvant, un antigène, un facteur de stimulation ou d'inhibition immune, un facteur hématopoïétique, un produit anti-cancer, un agent anti-inflammatoire, un composé anti-parasitaire, un agent antimicrobien, un fragment d'acide nucléique, un plasmide, un vecteur ADN, un activateur ou inhibiteur de prolifération cellulaire, un facteur de différentiation cellulaire, un facteur de coagulation sanguine, une immunoglobuline, un produit anti-angiogénique, un marqueur de sélection négative ou agent "suicide", un composé toxique, un agent anti-angiogénique, un polypeptide, un agent anti-cancer, des médicaments de production d'acide, et un antagoniste du récepteur histamine H2.

4. La composition selon la revendication 1, **caractérisée en ce que** ledit agent de neutralisation est présent en quantité suffisante pour neutraliser la dégradation acide dans le système digestif dudit animal et permettre la livraison dudit agent physiologiquement actif au niveau de l'intestin dudit animal.

5. La composition selon la revendication 1, **caractérisée en ce que** ledit agent de neutralisation est choisi parmi le groupe comprenant des anti-acides, du bicarbonate de sodium, du carbonate de sodium, du citrate de sodium, de l'hydrogénocarbonate de sodium, du phosphate de calcium, du carbonate de calcium, des sels de magnésium, du carbonate de magnésium, du trisilicate de magnésium, de l'hydroxyde de magnésium, du phosphate de magnésium, de l'oxyde de magnésium, du subcarbonate de bismuth et des combinaisons de ceux-ci.

6. La composition selon la revendication 5, **caractérisée en ce que** ledit agent de neutralisation est au moins le carbonate de sodium à une concentration de 10% à 20% m/m, ou le carbonate de calcium à une concentration de 10% à 20% m/m de ladite composition.

7. La composition selon la revendication 1, **caractérisée en ce que** ledit inhibiteur est en quantité suffisante pour inhiber la dégradation dudit agent physiologiquement actif par les enzymes de digestion dans le système digestif dudit animal et permettre la livraison dudit agent physiologiquement actif au niveau de l'intestin dudit animal.

8. La composition selon la revendication 1, **caractérisée en ce que** ledit inhibiteur des enzymes de digestion est choisi parmi le groupe comprenant une anti-protéase, de l'albumine d'oeuf, des inhibiteurs dérivés de plantes provenant de graines oléagineuses, de fève de soja, d'haricot, de fève faba, de son de riz et de son de blé, d'éthylène diamine tétra acétate, de l'alpha-1 antitrypsine, de l'albumine, de l'ovalbumine, et des protéosomes.

9. La composition selon la revendication 8, **caractérisée en ce que** ledit inhibiteur comprend au moins un inhibiteur de pepsine ou un inhibiteur d'entéropeptidase.

10. La composition selon la revendication 8, **caractérisée en ce que** ledit inhibiteur est l'albumine à une concentration entre 10% et 20% m/m.

11. La composition selon la revendication 1, **caractérisée en ce que** ledit agent augmentant la capture est choisi parmi le groupe comprenant des sels biliaires, de la saponine, du désoxycholate, du salicylate de sodium, du lauryl sulfate de sodium, de l'acide oléique, de l'acide linoléique, du monooléine, de la lécithine, de la lysolécithine, du polyoxyéthylène ester de sorbitan, du p-t-octylphénoxypolyoxyéthylene, du N-lauryl-p-D-maltopyranoside, du 1 dodecylazacycloheptane-2-azone, et d'un phospholipide.

12. La composition selon la revendication 11, **caractérisée en ce que** ledit agent augmentant la capture est le désoxycholate à une concentration entre 1% et 5%.

13. La composition selon la revendication 1, comprenant en plus au moins un ingrédient additionnel choisi parmi le groupe comprenant un éthylène diamine tétra acétate, un agent de conservation, un antioxydant, un colorant, un liant, un traceur, un édulcorant, un surfactant, un agent de démoulage, un aromatisant, de la farine brute, une fève, de la levure, de la levure de bière, de l'huile minérale, de l'huile végétale, de l'huile animale, un lubrifiant, un onguent, et des combinaisons de ceux-ci.

14. La composition selon la revendication 3, **caractérisée en ce que** ledit agent physiologiquement actif lorsque livré audit intestin de l'humain ou l'animal est absorbé par ledit intestin pour une livraison systémique.

15. La composition selon la revendication 3, **caractérisée en ce que** ledit agent physiologiquement actif lorsque livré audit intestin de l'humain ou l'animal a un effet physiologique sur la paroi intestinale.

16. La composition selon la revendication 3, **caractérisée en ce que** ledit agent physiologiquement actif lorsque livré audit intestin de l'humain ou l'animal a un effet physiologique sur le contenu dudit intestin.

17. La composition selon la revendication 1, **caractérisée en ce que** ledit animal est un oiseau, un mammifère, un insecte, ou un poisson.

18. La composition selon la revendication 3, **caractérisée en ce que** ledit agent physiologiquement actif est apte à induire une réponse immune contre les maladies infectieuses des muqueuses chez ledit humain ou animal.

19. Un médicament comprenant la composition selon l'une des revendications précédentes.

20. Un médicament comprenant une quantité suffisante d'une composition selon la revendication 3, 14, 15 ou 16.

21. Le médicament selon la revendication 20, pour la livraison systémique d'un agent physiologiquement actif à un humain ou un animal.

22. Le médicament selon la revendication 20, **caractérisé en ce que** ledit agent physiologiquement actif est un agent antimicrobien.

23. Le médicament selon l'une des revendications 19 à 22 pour le traitement des infections microbiennes des intestins d'un humain ou d'un animal.

24. Le médicament selon la revendication 23, **caractérisé en ce que** lesdites infections microbiennes sont causées par des microorganismes choisis parmi le groupe comprenant des bactéries, champignons, levures, virus, *Staphylococci, Streptococci, Micrococci, Peptococci, Peptostreptococci, Enterococci, Bacillus, Clostridium, Lactobacillus, Listeria, Erysipelothrix, Propionibacterium, Eubacterium, Corynebacterium, Mycoplasma, Ureaplasma, Streptomyces, Haemophilus, Nesseria, Eikenellus, Moraxellus, Actinobacillus, Pasteurella, Bacteroides, Fusobacteria, Prevotella, Porphyromonas, Veillonella, Treponema, Mitsuokella, Capnocytophaga, Campylobacter, Klebsiella, Chlamydia,* et des coliformes.

25. Le médicament selon la revendication 22, **caractérisé en ce que** ledit agent antimicrobien est choisi parmi le groupe comprenant des antibiotiques, bactériocines, lantibiotiques, probiotiques, antifongiques, antimycotiques, antiparasitaires, aminoglycosides, vancomycine, rifampine, lincomycine, chloramphénicol, fluoroquinol, pénicilline, bêta-lactames, amoxicilline, ampicilline, azlocilline, carbenicilline, mezlocilline, nafcilline, oxacilline, pipéracilline, ticarcilline, céftazidime, céftizoxime, céftriaxone, céfuroxime, céphalexine, céphalothine, imipénène, aztréonam, gentamicine, nétilmycine, tobramycine, tetracyclines, sulfonamides, macrolides, érythromycine, clarithromycine, azithromycine, polymyxine B, et clindamycine antibiotique.

26. Un médicament comprenant une quantité physiologiquement efficace d'une composition selon la revendication 3, pour augmenter l'absorption intestinale dudit agent physiologiquement actif chez l'humain ou l'animal.

27. Utilisation d'une composition selon l'une des revendications 1 à 18 pour la fabrication d'un médicament pour augmenter l'absorption intestinale dudit agent physiologiquement actif chez l'humain ou l'animal.
